(19) Europäisches Patentamt
European Patent Office
Office européen des brevets

(11) Veröffentlichungsnummer: **0 530 505 B1**

(12) **EUROPÄISCHE PATENTSCHRIFT**

(45) Veröffentlichungstag der Patentschrift: **11.10.95**

(51) Int. Cl.[6]: **C07K 5/06**, C07K 5/08, C07K 5/10, C07D 233/76, C07D 233/96, A61K 38/04

(21) Anmeldenummer: **92113086.0**

(22) Anmeldetag: **31.07.92**

# (54) Hydantoinderivate.

(30) Priorität: **08.08.91 DE 4126277**

(43) Veröffentlichungstag der Anmeldung:
**10.03.93 Patentblatt 93/10**

(45) Bekanntmachung des Hinweises auf die Patenterteilung:
**11.10.95 Patentblatt 95/41**

(84) Benannte Vertragsstaaten:
**AT BE CH DE DK ES FR GB GR IT LI LU NL SE**

(56) Entgegenhaltungen:
**EP-A- 0 449 079**

**CHEMICAL ABSTRACTS, vol. 103, no. 25, 23. Dezember 1985, Columbus, Ohio, US; abstract no. 215779s, B SCHWENZER ET AL. 'cleavage of Z-group and formation of hydantoin during alkaline saponification of Z-peptide esters' Seite 943-944 ;**

(73) Patentinhaber: **CASSELLA Aktiengesellschaft
Hanauer Landstrasse 526
D-60386 Frankfurt (DE)**

(72) Erfinder: **König, Wolfgang, Dr.
Eppsteiner Strasse 25
W-6238 Hofheim (DE)**
Erfinder: **Zoller, Gerhard, Dr.
Höhenstrasse 8
W-6368 Schöneck (DE)**
Erfinder: **Just, Melittta, Dr.
Theodor-Heuss-Strasse 80
W-6070 Langen (DE)**
Erfinder: **Jablonka, Bernd, Dr.
Dachbergstrasse 19a
W-6232 Bad Soden (DE)**

(74) Vertreter: **Urbach, Hans-Georg, Dr. et al
CASSELLA AKTIENGESELLSCHAFT,
Patentabteilung,
Hanauer Landstrasse 526
D-60386 Frankfurt (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 530 505 B1

## Beschreibung

Hydantoinderivate sind in der noch nicht veröffentlichten DE-P 4009506.1 (HOE 90/F 096) beschrieben. In weiterer Ausgestaltung fanden wir, daß auch Hydantoine der allgemeinen Formel I die Blutplättchenaggregation hemmen.

Gegenstand der Erfindung sind daher Verbindungen der Formel I,

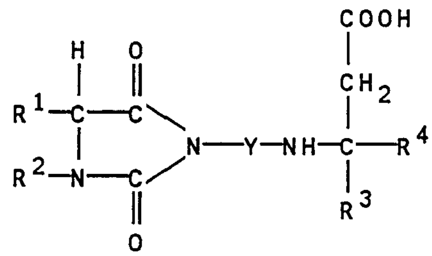

(I)

in welcher
Y $-(CH_2)_m-CO-$ oder

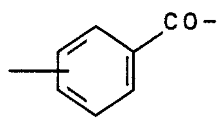

bedeutet und m für 1 - 4 steht;
$R^1$ für $-(CH_2)_n-NH-X$, $-CH_2-C_6H_4-NH-X$, $-CH_2-C_6H_4-C(=NH)-NH_2$, $-CH_2-C_6H_4-CH_2-NH-X$ oder $-C_6H_4-NH-X$ steht oder

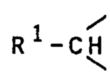

auch $>C=CH-C_6H_4-X^1$
bedeutet,
wobei n für eine ganze Zahl 3 - 5 steht,
$X^1$ $-CH_2NHX$, $-NHX$ oder $-C(=NH)-NH_2$ bedeutet,
X für Wasserstoff, $C_1$-$C_6$-Alkyl oder für einen Rest der Formel II steht,

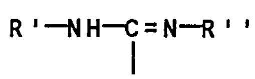

(II)

worin
R' und R'' unabhängig voneinander Wasserstoff oder $C_1$-$C_6$-Alkyl bedeuten;
$R^2$ Wasserstoff oder $C_1$-$C_6$-Alkyl bedeutet;
$R^3$ Wasserstoff oder einen Phenylrest bedeutet;
$R^4$ Wasserstoff, $COOR^5$, $CO-N(CH_3)-R^5$ oder $CO-NH-R^5$ bedeutet, worin
$R^5$ Wasserstoff oder unsubstituiertes oder ein- oder mehrfach durch gleiche oder verschiedene Reste $R^6$ substituiertes $C_1$-$C_8$-Alkyl bedeutet;
$R^6$ Hydroxy, Carboxy, Carboxamido, Amino, Mercapto, $C_1$-$C_8$-Alkoxy, $C_1$-$C_{18}$-Alkoxycarbonyl, Phenyl-$C_1$-$C_3$-alkoxycarbonyl, $C_3$-$C_8$-Cycloalkyl, Halogen, Nitro, Trifluormethyl, Phenyl, Phenyl-$C_1$-$C_8$-alkyl, $-NR^7R^8$, $-OR^7$, $-SR^7$, $-CONR^7R^8$, $-COOR^7$, $-COSR^7$, eine Aminosäureseitenkette, einen natürlichen oder unnatürlichen Aminosäure-, Iminosäure- oder einen Dipeptid-Rest sowie deren Ester und Amide bedeutet, wobei freie funktionelle Gruppen gegebenenfalls durch in der Peptidchemie üblichen Schutzgruppen geschützt sein können;

$R^7$ Wasserstoff, gegebenenfalls durch eine Aminogruppe substituiertes Phenyl-$C_1$-$C_8$-alkyl, $C_1$-$C_{18}$-Alkylcarbonyl, $C_1$-$C_8$-Alkyloxycarbonyl, Phenylcarbonyl, Phenyl-$C_1$-$C_8$-alkylcarbonyl, Phenyl-$C_1$-$C_8$-alkyloxycarbonyl, einen natürlichen oder unnatürlichen Aminosäure-, Iminosäure- oder einen Dipeptid-Rest bedeutet;
$R^8$ Wasserstoff, $C_1$-$C_8$-Alkyl, Phenyl oder Phenyl-$C_1$-$C_8$-alkyl bedeutet;
sowie deren physiologisch verträgliche Salze, wobei Verbindungen der Formel I ausgenommen sind, worin
$R^1$ $-(CH_2)_{3-4}$ - NH - X mit
X = $C_1$-$C_6$-Alkyl oder einen Rest der Formel II;
$R^2$ und $R^3$ Wasserstoff;
$R^4$ -CO-NH-$R^5$ und
Y $-CH_2$-CO bedeuten.

Alkyl kann geradkettig oder verzweigt sein. Entsprechendes gilt für davon abgeleitete Reste, wie z. B. Alkoxy, Alkanoyl und Phenylalkyl.

Unter Cycloalkyl werden auch alkylsubstituierte Reste, wie z. B. 4-Methylcyclohexyl oder 2,3-Dimethylcyclopentyl verstanden.

Unter Phenylalkyl versteht man z. B. einen mit $C_1$-$C_8$-Alkyl verknüpften unsubstituierten oder substituierten Phenylrest, wie z. B. Benzyl, Halobenzyl und Alkoxybenzyl, wobei Phenylalkyl jedoch nicht auf die genannten Reste beschränkt wäre.

Halogen steht für Fluor, Chlor, Brom oder Jod, insbesondere für Fluor oder Chlor.

Natürliche und unnatürliche Aminosäuren können, falls chiral, in der D- oder L-Form vorliegen. Bevorzugt sind $\alpha$-Aminosäuren. Beispielsweise seien genannt (vgl. Houben-Weyl, Methoden der organischen Chemie, Band XV/1 und 2, Stuttgart, 1974):
Aad, Abu Abu, ABz, 2ABz, $\epsilon$Aca, Ach, Acp, Adpd, Ahb, Aib, $\beta$Aib, Ala $\beta$Ala, Ala, Alg, All, Ama, Amt, Ape, Apm, Apr, Arg, Asn, Asp, Asu, Aze, Azi, Bai, Bph, Can, Cit, Cys, $(Cys)_2$, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hIle, hLeu, hLys, hMet, hPhe, hPro, hSer, hThr, hTrp, hTyr, Hyl, Hyp, 3Hyp, Ile, Ise, Iva, Kyn, Lant, Lcn, Leu, Lsg, Lys, $\beta$Lys, Lys, Met, Mim, Min, nArg, Nle, Nva, Oly, Orn, Pan, Pec, Pen, Phe, Phg, Pic, Pro, Pro, Pse, Pya, Pyr, Pza, Qin, Ros, Sar, Sec, Sem, Ser, Thi, $\beta$Thi, Thr, Thy, Thx, Tia, Tle, Tly, Trp, Trta, Tyr, Val, Tbg, Npg, Chg, Cha, Thia, 2,2-Diphenylaminoessigsäure, 2-(p-Tolyl)-2-phenylaminoessigsäure, 2-(p-Chlorphenyl)-aminoessigsäure.

Unter Aminosäureseitenketten werden Seitenketten von natürlichen oder unnatürlichen Aminosäuren verstanden.

Als Rest einer Iminosäure kommen insbesondere Reste von Heterocyclen aus der folgenden Gruppe in Betracht:
Pyrrolidin-2-carbonsäure; Piperidin-2-carbonsäure; Tetrahydroisochinolin-3-carbonsäure; Decahydroisochinolin-3-carbonsäure; Octahydroindol-2-carbonsäure; Decahydrochinolin-2-carbonsäure; Octahydrocyclopenta[b] pyrrol-2-carbonsäure; 2-Aza-bicyclo-[2.2.2]octan-3-carbonsäure; 2-Azabicyclo-[2.2.1]heptan-3-carbonsäure; 2-Azabicyclo[3.1.0]hexan-3-carbonsäure; 2-Azaspiro[4.4]nonan-3-carbonsäure; 2-Azaspiro[4.5]decan-3-carbonsäure; Spiro(bicyclo[2.2.1]-heptan)-2,3-pyrrolidin-5-carbonsäure; Spiro-(bicyclo[2.2.2]octan)-2,3-pyrrolidin-5-carbonsäure; 2-Azatricyclo[$4.3.0.1^{6,9}$]decan-3-carbonsäure;Decahydrocyclohepta[b]pyrrol-2-carbonsäure; Decahydrocycloocta[c]pyrrol-2-carbonsäure; Octahydrocyclopenta[c]pyrrol; Octahydroisoindol-1-carbonsäure; 2,3,3a,4,6a-Hexahydrocyclopenta[b]pyrrol-2-carbonsäure; 2,3,3a,4,5,7a-Hexahydroindol-2-carbonsäure; Tetrahydrothiazol-4-carbonsäure; Isoxazolidin-3-carbonsäure; Pyrazolidin-3-carbonsäure; Hydroxyprolin-2-carbonsäure, die alle gegebenenfalls substituiert sein können;

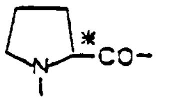 ; 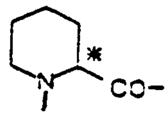 ;

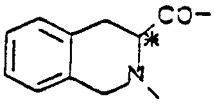 ; 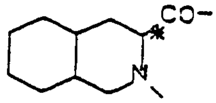 ; 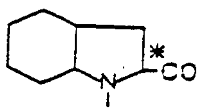 ; 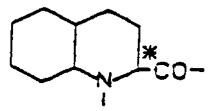 ;

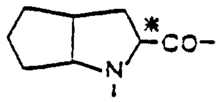 ; 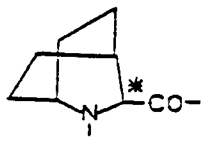 ; 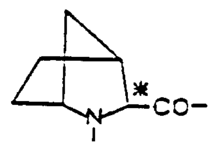 ; 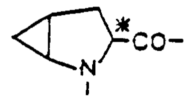 ;

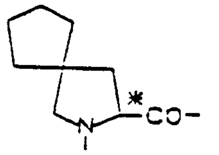 ; 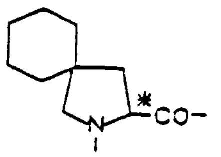 ; 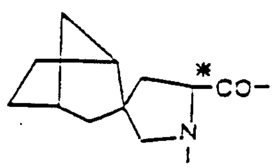 ;

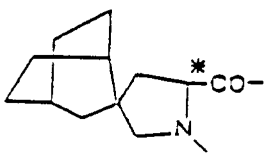 ; 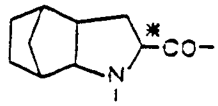 ; 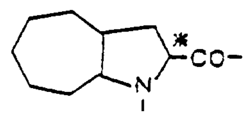 ;

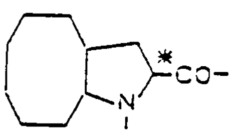 ; 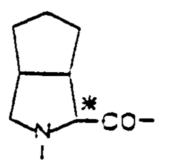 ; 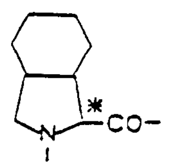 ;

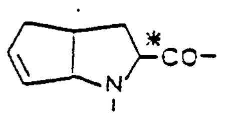 ; 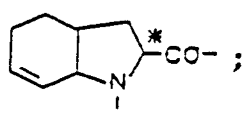 ; 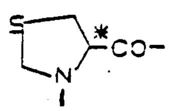 ;

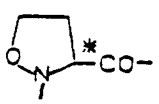 ; 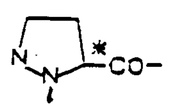 ; 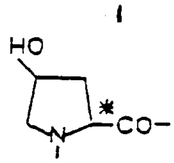 ;

Die oben genannten Reste zugrundeliegenden Heterocyclen sind beispielsweise bekannt aus US-A 4,344,949; US-A 4,374,847; US-A 4,350,704; EP-A 29,488; EP-A 31,741; EP-A 46,953; EP-A 49,605; EP-A 49,658; EP-A 50,800; EP-A 51,020; EP-A 52,870; EP-A 79,022; EP-A 84,164; EP-A 89,637; EP-A 90,341; EP-A 90,362; EP-A 105,102; EP-A 109,020; EP-A 111,873; EP-A 271,865 und EP-A 344,682.

Dipeptide können als Bausteine natürliche oder unnatürliche Aminosäuren, Iminosäuren sowie Azaaminosäuren enthalten. Ferner können die natürlichen oder unnatürlichen Aminosäuren, Iminosäuren und Dipeptide auch als Ester bzw. Amide vorliegen, wie z. B. Methylester Ethylamid, Semicarbazid, -Amino-$C_4$-$C_8$-alkylamid.

Funktionelle Gruppen der Aminosäuren, Iminosäuren und Dipeptide können geschützt vorliegen. Geeignete Schutzgruppen wie z. B. Urethanschutzgruppen, Carboxylschutzgruppen und Seitenkettenschutzgruppen sind bei Hubbuch, Kontakte (Merck) 1979, Nr. 3, Seiten 14 bis 23 und bei Büllesbach, Kontakte (Merck) 1980, Nr. 1, Seiten 23 bis 35 beschrieben. Insbesondere seien genannt: Aloc, Pyoc, Fmoc, Tcboc, Z, Boc, Ddz, Bpoc, Adoc, Msc, Moc, Z(NO2), Z(Haln), Bobz, Iboc, Adpoc, Mboc, Acm, tert.-Butyl, OBzl, ONbzl, OMbzl, Bzl, Mob, Pic, Trt.

Unter Salzen von Verbindungen der Formel (I) sind insbesondere pharmazeutisch verwendbare oder nicht-toxische Salze zu verstehen.

Solche Salze werden beispielsweise von Verbindungen der Formel (I), welche saure Gruppen, z. B. Carboxy, enthalten, mit Alkali- oder Erdalkalimetallen gebildet, wie z. B. Na, K, Mg und Ca, sowie mit physiologisch verträglichen organischen Aminen, wie z. B. Triethylamin und Tris-(2-hydroxy-ethyl)-amin.

Verbindungen der Formel (I), welche basische Gruppen, z. B. eine Aminogruppe oder ein Guanidinogruppe enthalten, bilden Salze mit anorganischen Säuren, wie z. B. Salzsäure, Schwefelsäure oder Phosphorsäure und mit organischen Carbon- oder Sulfonsäuren, wie z. B. Essigsäure, Citronensäure, Benzoesäure, Maleinsäure, Fumarsäure, Weinsäure und
p-Toluolsulfonsäure.

Bevorzugt sind Verbindungen der Formel I worin
$R^1$ $-CH_2-C_6H_4-C(NH)-NH_2$ oder $-CH_2-C_6H_4-CH_2-NH_2$,
$R^2$ H oder $CH_3$,
Y $-CH_2-CO-$ und
$R^4$ $-CO-NH-R^5$ bedeuten, wobei $NH-R^5$ für einen $\alpha$-Aminosäurerest, bevorzugt einen Valin- oder Phenylglycin-Rest, steht.

Verbindungen der Formel I werden hergestellt durch Fragmentkondensation von z. B. Verbindungen der Formel IIIa, IIIb oder IIIc mit Verbindungen der Formel IV, wobei $R^1$, $R^2$, $R^3$, $R^4$, $X^1$ und m die oben genannten Bedeutungen haben:

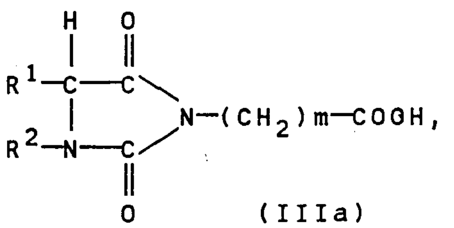

(IIIa)

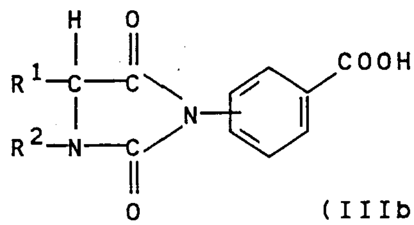

(IIIb)

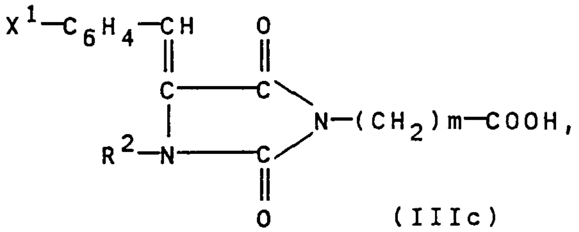

(IIIc)

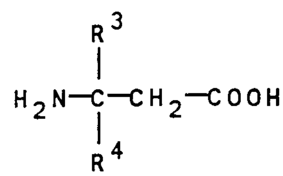

(IV)

Aminogruppen in $R^1$ und $R^4$ müssen durch reversible Schutzgruppen während der Kondensation geschützt werden. Auch die Carboxylgruppen in Verbindungen der Formel IV sollten während der Kondensation als Benzyl oder tert.-Butylester vorliegen. Ein Aminogruppen-Schutz erübrigt sich, wenn die zu generierenden Aminogruppen noch als Nitro- oder Cyanogruppen vorliegen und erst nach der Kupplung durch Hydrierung gebildet werden. Nach der Kupplung werden die vorhandenen Schutzgruppen geeignet abgespalten. $NO_2$-Gruppen (Guanidinoschutz), Benzyloxycarbonylgruppen und Benzylester werden abhydriert. Die Schutzgruppen vom tert.-Butyltyp werden sauer gespalten. Der 9-Fluorenylmethyloxycarbonylrest wird durch sekundäre Amine entfernt. Zur
Kupplung von z. B. Verbindungen der Formeln IIIa/IIIb/IIIc mit Verbindungen der Formel IV verwendet man die Kondensationsmethoden der Peptidchemie.

Hydantoine der Formel Va entstehen ganz allgemein durch basische Behandlung von Alkyloxycarbonyl oder Aralkyloxycarbonyl-Peptiden der allgemeinen Formel V (J. S. Fruton und M. Bergmann, J. Biol. Chem. 145 (1942) 253 - 265; C. A. Dekker, S. P. Taylor, jr. und J. S. Fruton, J. Biol. Chem. 180 (1949) 155 - 173; M. E. Cox, H. G. Garg, J. Hollowood, J. M. Hugo, P. M. Scopes and G. T. Young, J. Chem. Soc (1965) 6806 - 6813; W. Voelter und A. Altenburg, Liebigs Ann. Chem. (1983) 1641 - 1655; B. Schwenzer, E. Weber und G. Losse, J. Prakt. Chem. 327 (1985) 479 - 486):

$R^{10}-O-CO-NH-CHR^{11}-CO-NH-CH_2-CO-R^{12}$ (V)

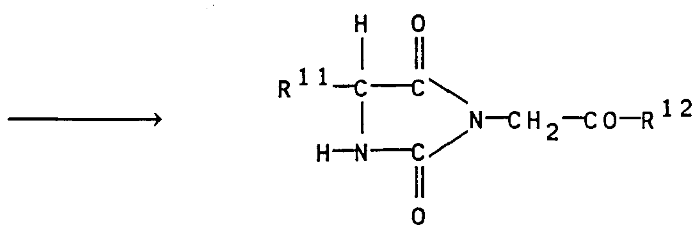

(Va)

worin $R^{10}$ Benzyl oder tert.-Butyl, $R^{11}$ eine beliebige Aminosäureseitenkette und $R^{12}$ ein Amid, einen Aminosäure- oder einen Peptid-Rest bedeuten. Dabei racemisiert jedoch die N-terminale Aminosäure und das Hydantoin hydrolysiert in das Harnstoffderivat

$HOOC-CH(R^{11})-NH-CO-NH-CH2-CO-R^{12}$

(W. Voelter und A. Altenburg, Liebigs Ann. Chem. (1983) 1641 - 1655).

Eine milde Methode ist dagegen die Zyklisierung zu den Hydantoinen aus Verbindungen der Formel V unter neutralen Bedingungen durch Behandlung mit Tetrabutylammoniumfluorid in Tetrahydrofuran unter Rückfluß (J. Pless, J. Org. Chem. 39 (1974) 2644 - 2646).

Eine weitere Möglichkeit einer milden Zyklisierung ist die Trimethylsilylierung der Peptidbindung zwischen der N-terminalen Aminosäure und dem folgenden Glycin mit Bistrimethylsilyltrifluoroacetamid in Acetonitril (4 Stunden unter Rückfluß) (J. S. Davies, R. K. Merritt und R. C. Treadgold, J. Chem. Soc. Perkin Trans. I (1982) 2939 - 2947).

In der älteren Anmeldung DE-P 4009506.1 (HOE 90/F 096) ist beschrieben, daß Peptide der Formel Vb nach längerer Zeit bereits bei Raumtemperatur oder mit Tetrahydrofuran kurz im Rückfluß gekocht, zu den Hydantoin-Derivaten zyklisieren.

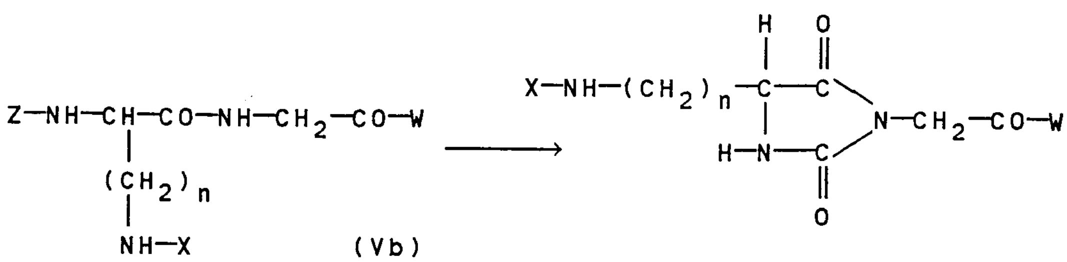

(Vb)

wobei
Z Benzyloxycarbonyl,
X = Formamidino und
W OtBu, OBzl, Asp(OtBu)-NH-R5 bedeuten und mögliche Carboxylgruppen als Ester, vorzugsweise OtBu oder OBzl, vorliegen.

Durch Kondensation von Aminosäuren, N-Alkylaminosäuren oder bevorzugt deren Ester (z. B. Methyl-, Ethyl-, Benzyl- oder tert.-Butylestern) der Formel VI mit Isocyanatoalkancarbonsäureestern erhält man Harnstoffderivate der Formel VII, die durch Erhitzen in Salzsäure unter Verseifung der Esterfunktionen zu Hydantoinderivaten der Formel IIIa zyklisieren (siehe DE-P 4009506.1). Während der Harnstoffsynthese können Guanidinogruppen durch Schutzgruppen (z. B. $NO_2$ oder Mtr) blockiert werden. Mögliche Aminogruppen in der Seitenkette müssen bei der Harnstoffsynthese in geschützter Form (z. B. als Boc-oder Z-Derivate) oder noch als $NO_2$ oder Cyanofunktion vorliegen, die später zur Aminogruppe reduziert oder im Falle der Cyanogruppe auch in die Formamidinogruppe umgewandelt werden, wie z.B.

$R^2-NH-CH-(R^1)COOCH_3 + O=C=N-(CH_2)_m-COOC_2H_5$ (VI)

$$\xrightarrow{6N\ HCl} C_2H_5OOC-(CH_2)_m-NH-CO-NH(R^2)-CH(R^1)-COOCH_3 \longrightarrow IIIa$$

(VII)

Die Verbindungen der Formel IIIb erhält man analog, wenn man statt Isocyanatoalkancarbonsäureester die Isocyanate der Amino-benzoesäureester verwendet.

Eine weitere Möglichkeit zu den Hydantoinen der Formel IIIa zu kommen, sind die Hydantoine der Formel VIII, die am Imidstickstoff mit Halogenalkancarbonsäuren oder deren Ester (z. B. Alkyl- oder Aralkylester) alkyliert und an der $CH_2$-Funktion mit geeigneten Aldehyden kondensiert werden können (Gränacher und Landolt, Helv. Chim. Acta 10 (1927) 808). Durch Hydrierung der Kondensationsprodukte kommt man zu den erfindungsgemäßen Ausgangsprodukten der Formel IIIa. Wird die Hydrierung erst nach der Kondensation mit Verbindungen der Formel IV durchgeführt, so erspart man sich den Schutz der Aminogruppe.

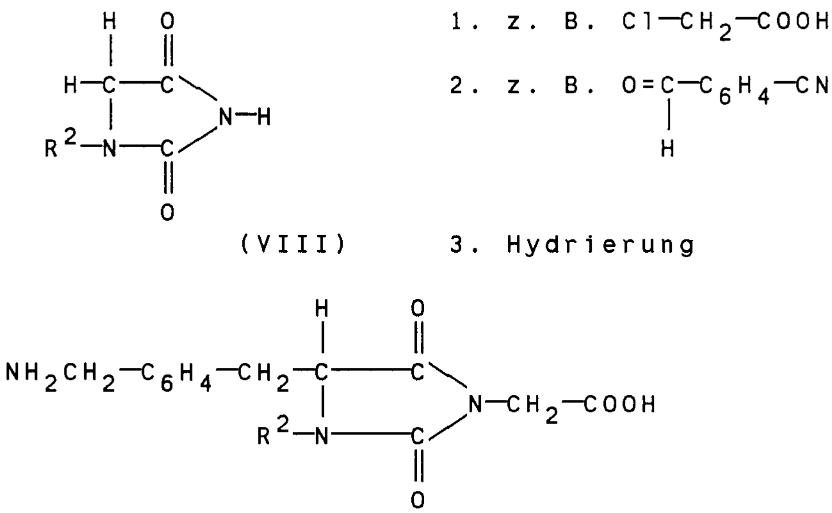

(VIII)

Die Hydantoine der Formel IIIa mit $R^2$ = Alkyl können auch auf folgendem Weg hergestellt werden: Der unsubstituierte Hydantoin-Grundkörper wird zuerst mit Halogenalkancarbonsäuren oder deren Ester am Iminostickstoff alkyliert. Um $R^1$ einzuführen, kondensiert man das so erhaltene Hydantoin mit geeigneten Aldehyden. Danach alkyliert man gegebenenfalls mit Alkylhalogeniden den 2. Stickstoff (D. A. Hahn und J. Evans, J. Amer. Chem. Soc. 50 (1928) 806 -818) und hydriert, gegebenenfalls erst nach der Kondensation mit Verbindungen der Formel IV, die Doppelbindung und mögliche Nitrogruppen oder Cyanogruppen in einem Schritt. Hat man bei der Kondensation die Reste

$>CH-C_6H_4-X^2$

($X^2$ = Cyano oder Acetylamino) eingeführt, so kann man ohne zu hydrieren den Rest $X^2$ in den Rest $X^1$ überführen und kommt so zu den Verbindungen der allgemeinen Formel IIIc.

Die Guanylierung der Aminofunktionen kann mit folgenden Reagentien durchgeführt werden:

1. O-Methylisothioharnstoff (S. Weiss und H. Krommer, Chemiker Zeitung 98 (1974) 617 - 618),
2. S-Methylisothioharnstoff (R. F. Borne, M. L. Forrester und I. W. Waters, J. Med. Chem. 20 (1977) 771 - 776),
3. Nitro-S-methylisothioharnstoff (L. S. Hafner und R. E. Evans, J. Org. Chem. 24 (1959) 1157),
4. Formamidinsulfonsäure (K. Kim, Y.-T. Lin und H. S. Mosher, Tetrah. Lett. 29 (1988) 3183 - 3186),
5. 3,5-Dimethyl-1-pyrazolyl-formamidinium-nitrat (F. L. Scott, D. G.

O'Donovan und J. Reilly, J. Amer. Chem. Soc. 75 (1953) 4053 - 4054).

Die Herstellung von Formamidinen aus den entsprechenden Cyano-Verbindungen kann durch Anlagerung von Methanol oder Ethanol in saurem wasserfreien Medium (z.B. Dioxan, Ethanol oder Methanol) und anschließender Behandlung mit Ammoniak in Ethanol oder Isopropanol durchgeführt werden (G. Wagner, P. Richter und Ch. Garbe, Pharmazie 29 (1974) 12 - 55). Eine weitere Methode, Formamidine herzustellen, ist die Analgerung von $H_2S$ an die Cyanogruppe, gefolgt von einer Methylierung des entstandenen Thioamids und anschließender Umsetzung mit Ammoniak (DDR-Patent Nr. 235 866).

Die Ausgangspeptide der Formel IV werden in der Regel vom C-terminalen Ende her stufenweise aufgebaut. Die Peptidknüpfungen können mit den bekannten Kupplungsmethoden der Peptidchemie durchgeführt werden.

Die Verbindungen der allgemeinen Formel I und ihre physiologisch verträglichen Salze können als Heilmittel für sich allein, in Mischungen untereinander oder in Form von pharmazeutischen Zubereitungen verabreicht werden, die eine enterale oder parenterale Anwendung gestatten und die als aktiven Bestandteil eine wirksame Dosis mindestens einer Verbindung der allgemeinen Formel I oder eines Salzes davon, neben üblichen pharmazeutisch einwandfreien Träger- und Zusatzstoffen enthalten. Die Zubereitungen enthalten normalerweise etwa 0,5 bis 90 Gew% der therapeutisch wirksamen Verbindung.

Die Heilmittel können oral, z. B. in Form von Pillen, Tabletten, Lacktabletten, Dragees, Granulaten, Hart- und Weichgelatinekapseln, Lösungen, Sirupen, Emulsion oder Suspensionen oder Aerosolmischungen verabreicht werden. Die Verabreichung kann aber auch rektal, z. B. in Form von Suppositorien oder parenteral, z. B. in Form von Injektionslösungen oder Mikrokapseln, perkutan, z. B. in Form von Salben oder Tinkturen, oder nasal, z. B. in Form von Nasalsprays, erfolgen.

Die Herstellung der pharmazeutischen Präparate erfolgt in an sich bekannter Weise, wobei pharmazeutisch inerte anorganische oder organische Trägerstoffe verwendet werden. Für die Herstellung von Pillen, Tabletten, Dragees und Hartgelatinekapseln kann man z. B. Lactose, Maisstärke oder Derivate davon, Talk, Stearinsäure oder deren Salze etc. verwenden. Trägerstoffe für Weichgelatinekapseln und Suppositorien sind z. B. Fette, Wachse, halbfeste und flüssige Polyole, natürliche oder gehärtete Öle etc. Als Trägerstoffe für die Herstellung von Lösungen und Sirupen eignen sich z. B. Wasser, Saccharose, Invertzucker, Glukose, Polyole etc. Als Trägerstoffe für die Herstellung von Injektionslösungen eignen sich Wasser, Alkohole, Glycerin, Polyole, pflanzliche Öle etc. Als Trägerstoffe für Mikrokapseln oder Implantate eignen sich Mischpolymerisate aus Glykolsäure und Milchsäure.

Die pharmazeutischen Präparate können neben den Wirk- und Trägerstoffen noch Zusatzstoffe, wie z. B. Füllstoffe, Streck-, Spreng-, Binde-, Gleit-, Netz-, Stabilisierungs-, Emulgier-, Konservierungs-, Süß-, Färbe-, Geschmacks- oder Aromatisierungs-, Dickungs-, Verdünnungsmittel, Puffersubstanzen, ferner Lösungsmittel oder Lösungsvermittler oder Mittel zur Erzielung eines Depoteffekts, sowie Salze zur Veränderung des osmotischen Drucks, Überzugsmittel oder Antioxidantien enthalten. Sie können auch zwei oder mehrere Verbindungen der allgemeinen Formel I oder ihrer physiologisch verträglichen Salze und noch einen oder mehrere andere therapeutisch wirksame Stoffe enthalten.

Derartige andere therapeutisch wirksame Substanzen sind beispielsweise durchblutungsfördernde Mittel, wie Dihydroergocristin, Nicergolin, Buphenin, Nicotinsäure und ihre Ester, Pyridylcarbinol, Bencyclan, Cinnarizin, Naftidrofuryl, Raubasin und Vincamin; positiv inotrope Verbindungen, wie Digoxin, Acetyldigoxin, Metildigoxin und Lantano-Glykoside; Coronardilatatoren, wie Carbochromen; Dipyridamol, Nifedipin und Perhexilin; antianginöse Verbindungen, wie Isosorbiddinitrat, Isosorbidmononitrat, Glycerolnitrat, Molsidomin und Verapamil; β-Blocker, wie Propranolol, Oxprenolol, Atenolol, Metoprolol und Penbutolol. Darüberhinaus lassen sich die Verbindungen mit anderen nootrop wirksamen Substanzen, wie z. B. Piracetam, oder ZNS-aktiven Substanzen, wie Pirlindol, Sulpirid etc., kombinieren.

Die Dosis kann innerhalb weiter Grenzen variieren und ist in jedem einzelnen Fall den individuellen Gegebenheiten anzupassen. Im allgemeinen ist bei der oralen Verabreichung eine Tagesdosis von etwa 0,1 bis 1 mg/kg, vorzugsweise 0,3 bis 0,5 mg/kg Körpergewicht zur Erzielung wirksamer Ergebnisse angemessen, bei intravenöser Applikation beträgt die Tagesdosis im allgemeinen etwa 0,01 bis 0,3 mg/kg, vorzugsweise 0,05 bis 0,1 mg/kg Körpergewicht. Die Tagesdosis wird normalerweise, insbesondere bei der Applikation größerer Mengen, in mehrere, z. B. 2, 3 oder 4 Teilverabreichungen aufgeteilt. Gegebenenfalls kann es, je nach individuellem Verhalten, erforderlich werden, von der angegebenen Tagesdosis nach oben oder nach unten abzuweichen. Pharmazeutische Präparate enthalten normalerweise 0,2 bis 50 mg, vorzugsweise 0,5 bis 10 mg Wirkstoff der allgemeinen Formel I oder eines ihrer physiologisch verträglichen Salze pro Dosis.

Die erfindungsgemäßen Verbindungen haben die Fähigkeit die Zell-Zell-Adhäsion zu hemmen, die auf Interaktionen von Arg-Gly-Asp-enthaltenden Glykoproteinen mit den sogenannten Integrinen beruht. Integrine sind Transmembran-Glykoproteine, Rezeptoren für Arg-Gly-Asp-enthaltenden Zellmatrix-Glykoproteine (E. Ruoslahti und M. D. Pierschbacher, Science 238 (1987) 491 - 497; D. R. Phillips, I. F. Charo, L. V. Parise und L. A. Fitzgerald, Blood 71 (1988) 831 - 843).

Die erfindungsgemäßen neuen Hydantoin-Derivate der Formel I hemmen die Thrombozytenaggregation, die Metastasierung sowie die Osteoclastenbindung an die Knochenoberflächen.

Eine akute Anwendung finden die Hydantoin-Derivate der Formel I daher bei Thrombosegefahr und der Gefahr einer Reocclusion bei Herzinfarkt; eine chronische Anwendung bei der Prävention der Arteriosklerose und Thrombose. Weitere Anwendung ist während Krebsoperationen und auch prophylaktisch bei Krebs gegeben. Ferner kann Osteoporose durch Hemmung der Osteoclastenbindung an die Knochenoberfläche vermieden werden.

Geprüft werden die Verbindung vor allem auf ihre hemmende Wirkung bei der Blutplättchenaggregation und der Anhaftung von Fibrinogen an Blutplättchen.
Verwendet werden gelfiltrierte Blutplättchen aus humanem Spenderblut, die mit ADP oder Thrombin aktiviert werden.

**Beispiele**

Alle Produkte wurden über Massenspektren und NMR-Spektren identifiziert.

Beispiel 1:

[5-(S,R)-(4-Formamidino-benzyl)-2,4-dioxoimidazolidin-3-yl]-acetyl-L-aspartyl-L-valin.

1 a. 4-Formamidino-D,L-phenylalanin-methylester-dihydrochlorid

11 g (39 mmol) 4-Formamidino-D,L-phenylalanin-dihydrochlorid werden in 110 ml Methanol suspendiert. Bei -10 °C läßt man 2,9 ml (39 mmol) Thionylchlorid zutropfen und eine Stunde bei Raumtemperatur und 45 min. bei 45 °C rühren.

Da sich noch nicht alles umgesetzt hat, werden erneut 1 ml Thionylchlorid bei -10 °C zugetropft. Man läßt anschließend 2 Stunden bei 40 °C rühren und übers Wochenende bei Raumtemperatur stehen. Danach wird im Vakuum eingeengt und der Rückstand mit Diethylether verrieben und abgesaugt. Ausbeute: 11,27 g.

Zur Abtrennung von Verunreinigungen wird jeweils ein Drittel der Substanz über ®Sephadex LH20 (200 x 4 cm) in Wasser chromatographiert. Die Fraktionen mit dem Methylester werden vereinigt und gefriergetrocknet. Ausbeute: 10,47 g (91 %), Schmelzpunkt: 166 °C.

1 b. N-[1-Methoxycarbonyl-2-(S,R)-(4-formamidino-phenyl)-ethyl]-N'-ethyl-oxycarbonylmethyl-harnstoff-hydrochlorid

Zu einer Lösung von 3,73 g (12,74 mmol) 4-Formamidino-D,L-phenylalanin-methylester-dihydrochlorid in 28 ml Dimethylformamid gibt man bei Raumtemperatur 1,4 ml (12,74 mmol) N-Ethylmorpholin und läßt innerhalb von 15 Minuten 1,44 ml (12,74 mmol) Isocyanatoessigsäureethylester langsam zutropfen. Man läßt 1 Stunde bei Raumtemperatur rühren und engt ein. Der ölige Rückstand wird wie in Beispiel 1a chromatographisch gereinigt und gefriergetrocknet. Ausbeute 3,82 g (77 %) amorphe Substanz.

1 c. [5-(S,R)-(4-Formamidino-benzyl)-2,4-dioxoimidazolidin-3-yl]-essigsäure

3,8 g (9,8 mmol) N-[1-Methoxycarbonyl-2-(S,R)-(4-formamidino-phenyl)-ethyl]-N'-ethyloxycarbonyl-methyl-harnstoff-hydrochlorid werden in 35 ml 6N HCl 30 Minuten unter Rückfluß gekocht. Die Lösung wird im Vakuum eingeengt und der Rückstand in 250 ml Wasser gelöst. Mit gesättigter $NaHCO_3$-Lösung wird die Lösung auf pH 5 eingestellt und auf 0 °C gekühlt. Der Niederschlag wird abgesaugt und über $P_2O_5$ im Vakuum getrocknet. Ausbeute 2,33 g, Schmelzpunkt 287 °C (unter Zersetzung). Die Mutterlauge wird eingeengt und mit ca. 20 ml Wasser versetzt.
Der ausfallende Niederschlag wird abgesaugt und wie oben getrocknet.
Ausbeute: 0,22 g.
Gesamtausbeute: 2,55 g (89 %).

1 d. [5-(S,R)-(4-Formamidino-benzyl)-2,4-dioxoimidazolidin-3-yl]-acetyl-Asp(OtBu)-Val-OtBu

Zu einer Suspension von 0,5 g [5-(S,R)-(4-Formamidinobenzyl)-2,4-dioxoimidazolidin-3-yl]-essigsäure, 0,583 g H-Asp(OtBu)-Val-OtBu•HCl und 207 mg HOBt in 5 ml Dimethylformamid gibt man bei 0 °C 340 mg (1,53 mmol) DCC. Man läßt 1 Stunde bei 0 °C und 4 Stunden bei Raumtemperatur rühren. Anschließend läßt man den Ansatz übers Wochenende im Kühlraum stehen, saugt den Niederschlag ab und engt das Filtrat ein. Zur Reinigung wird die Substanz über Kieselgel in Methylenchlorid/Methanol/ Wasser/Eisessig-Mischungen chromatographiert, z. B. 8 : 2 : 0, 2 : 0, 2.
Ausbeute 1,03 g amorphe Substanz (enthält noch Essigsäure).

1 e. [5-(S,R)-(4-Formamidino-benzyl)-2,4-dioxoimidazolidin-3-yl]-acetyl-L-aspartyl-L-valin

1,03 g [5-(S,R)-(4-Formamidino-benzyl)-2,4-dioxoimidazolidin-3-yl]-acetyl-Asp(OtBu)-Val-OtBu werden in eine Mischung von 9 ml Trifluoressigsäure, 1 ml Wasser und 1 ml Dimercaptoethan gelöst. Nach 1 Stunde bei Raumtemperatur wird mit Ether versetzt und die ausfallende Substanz abgesaugt. Die Substanz ist stark hygroskopisch. Zur Reinigung wird die Substanz an ®Sephadex LH20 in einer Mischung aus Eisessig, n-Butanol und Wasser chromatographiert. Die Fraktionen mit der reinen Substanz werden eingeengt. Der Rückstand wird in Wasser gelöst und gefriergetrocknet.
Ausbeute: 461,7 mg,
$[\alpha]_D^{25}$ = -14,9 ° (c = 1, in Essigsäure).

Beispiel 2:

[1-Methyl-5-(S)-(3-guanidino-propyl)-2,4-dioxoimidazolidin-3-yl]-acetyl-L-aspartyl-L-valin

2 a. N-Methyl-L-arginin-methylester-dihydrochlorid

Zu einer Suspension von 15,06 g N-Methylarginin in 50 ml abs. Methanol tropft man bei -10 °C 6,4 ml Thionylchlorid zu. Danach läßt man unter Rühren auf Raumtemperatur kommen. Da nach 17 Stunden Reaktionszeit noch Ausgangsmaterial vorhanden ist, gibt man portionsweise weitere 9,6 ml Thionylchlorid, erhitzt 4 Stunden auf 40 °C, saugt von Unlöslichem ab und engt das Filtrat ein. Der ölige Rückstand wird in Wasser gelöst, von Unlöslichem filtriert und gefriergetrocknet.
Ausbeute: 19,8 g amorphe Substanz.

2 b. N-Methyl-N-[1-methoxycarbonyl-2-(S)-(3-guanidino-propyl)-ethyl]-N'-ethyloxycarbonylmethyl-harnstoff-hydrochlorid

Zu einer Suspension von 2,4 g (10 mmol) N-Methyl-L-arginin-methylester-hydrochlorid in 10 ml Dimethylformamid gibt man bei Raumtemperatur 1,3 ml N-Ethylmorpholin und läßt sofort 1,13 ml Isocyanatoessigsäureethylester zutropfen. Nach 5 - 15 Minuten geht alles in Lösung. Nach etwa 2 Stunden wird der Ansatz im Hochvakuum eingeengt. Der Rückstand wird in Wasser gelöst und Unlösliches abfiltriert. Das Filtrat wird über ®Sephadex LH20 (200 x 4 cm) mit Wasser chromatographiert und die reinen Fraktionen gefriergetrocknet.
Ausbeute: 2,8 g amorphe, hygroskopische Substanz.

2 c. [1-Methyl-5-(S)-(3-guanidino-propyl)-2,4-dioxoimidazolidin-3-yl]-essigsäure-hydrochlorid

Die oben gewonnenen 2,8 g N-Methyl-N-[1-methoxycarbonyl-2-(S)-(3-guanidinopropyl)-ethyl]-N'-ethyloxycarbonylmethyl-harnstoff-hydrochlorid werden in 30 ml 6N HCl 30 Minuten im Rückfluß gekocht. Danach wird im Hochvakuum eingeengt, der Rückstand in Wasser gelöst und gefriergetrocknet.
Ausbeute 2,3 g.
Die Substanz wird zur Reinigung an ®Sephadex LH20 (200 x 4 cm) mit Wasser chromatographiert.
Ausbeute an reiner klebriger, amorpher Substanz: 1,5 g.

2 d. [1-Methyl-5-(S)-(3-guanidino-propyl)-2,4-dioxoimidazolidln-3-yl]-acetyl-Asp(OtBu)-Val-OtBu

Zu einer Lösung von 519,5 mg [1-Methyl-5-(S)-(3-guanidino-propyl)-2,4-dioxoimidazolidin-3-yl]-essigsäurehydrochlorid und 729 mg HCl•H-Asp(OtBu)-Val-OtBu und 258 mg HOBt in 5 ml Dimethylformamid gibt man bei 0 °C 0,25 ml N-Ethylmorpholin und 420 mg DCC. Man läßt 1 Stunde bei 0 °C und anschließend 3 Stunden rühren, läßt über Nacht bei Raumtemperatur stehen und saugt anderntags den Harnstoff ab. Das Filtrat wird eingeengt. Der Rückstand wird an Kieselgel mit Methylenchlorid/Methanol/Eisessig/Wasser-Mischungen chromatographiert. Die reinen Fraktionen werden vereinigt und eingeengt.
Ausbeute: 600 mg einer sehr hygroskopischen Substanz

2 e. [1-Methyl-5-(S)-(3-guanidino-propyl)-2,4-dioxoimidazolidin-3-yl]-acetyl-Asp-Val-OH

600 mg [1-Methyl-5-(S)-(3-guanidino-propyl)-2,4-dioxoimidazolidin-3-yl]-acetyl-Asp(OtBu)-Val-OtBu werden in 6 ml 90 %iger Trifluoressigsäure gelöst. Man läßt 1 Stunde bei Raumtemperatur stehen und engt dann ein. Der Rückstand wird in Wasser gelöst und gefriergetrocknet.
Ausbeute: ca. 500 mg einer klebrigen, hygroskopischen Substanz,
$[\alpha]_D^{23}$ = - 24 °(c = 1, in Wasser)

Beispiel 3:

[5-(S)-(4-Amino-butyl)-2,4-dioxoimidazolidin-3-yl]-acetyl-Asp-Val-OH

3 a. N-[1-Methoxycarbonyl-2-(4-benzyloxycarboxamido-butyl)-ethyl]-N'-ethyloxycarbonylmethyl-harnstoff

Zu einer Lösung von 6,61 g (20 mmol) H-Lys(Z)-OMe•HCl in 20 ml Dimethylformamid gibt man 2,6 ml N-Ethylmorpholin und tropft sofort 2,26 ml Isocyanatoessigsäureethylester zu. Die Lösung erwärmt sich dabei auf ca. 40 °C. Nach ca. 2 Stunden wird der Ansatz im Hochvakuum eingeengt. Der Rückstand wird zwischen Essigester und Wasser verteilt. Die Essigesterphase wird nacheinander mit gesättigter $NaHCO_3$-Lösung, $KHSO_4/K_2SO_4$-Puffer und Wasser ausgeschüttelt, über $Na_2SO_4$ getrocknet und eingeengt. Der Rückstand wird mit Petrolether verrieben, abgesaugt und getrocknet.
Ausbeute: 7,44 g,
Schmelzpunkt: 92 - 94 °C.

3 b. [5-(S)-(4-Amino-butyl)-2,4-dioxoimidazolidin-3-yl]-essigsäure-hydrochlorid

7,2 g N-[1-Methoxycarbonyl-2-(4-Benzyloxycarboxamido-butyl)-ethyl]-N'-ethyloxycarbonylmethyl-harnstoff werden in 63 ml 6N HCl 30 Minuten im Rückfluß erhitzt. Danach wird im Hochvakkum eingeengt und der Rückstand in Wasser gelöst. Von Unlöslichem wird filtriert und das Filtrat gefriergetrocknet.
Ausbeute 4,58 g amorphe hygroskopische Substanz.

3 c. [5-(S)-(4-Tert.-butyloxycarboxamido-butyl)-2,4-dioxoimidazolidin-3-yl]-essigsäure

Zu einer Lösung von 2,36 g [5-(S)-(4-Amino-butyl)-2,4-dioxoimidazolidin-3-yl]-essigsäure-hydrochlorid und 2,6 g $NaHCO_3$ in einer Mischung aus 10 ml Wasser und 20 ml Dioxan gibt man bei Raumtemperatur 2,5 g Di-tert.-butyldicarbonat. Da kaum Umsetzung eintritt, gibt man unter pH-Kontrolle 0,1N NaOH zu bis ein pH von 8 erreicht ist. Der Ansatz steht über Nacht. Die Lösung wird mit 1N HCl auf pH 6 eingestellt und eingeengt. Der Rückstand wird zwischen Essigester und
$KHSO_4/K_2SO_4$-Puffer verteilt. Die Essigesterphase wird über $Na_2SO_4$ getrocknet und eingeengt. Ausbeute: 2,92 g einer öligen Substanz.

3 d. [5-(S)-(4-Tert.-butyloxycarboxamido-butyl)-2,4-dioxoimidazolidin-3-yl]-acetyl-Asp(OtBu)-Val-OtBu

Zu einer Lösung von 820 mg [5-(S)-(4-tert.-butyloxycarboxamido-butyl)-2,4-dioxoimidazolidin-3-yl]-essigsäure, 948 mg HCl•H-Asp(OtBu)-Val-OtBu und 336 mg HOBt in 5 ml Dimethylformamid gibt man bei 0 °C, 0,325 ml N-Ethylmorpholin und 548 mg DCC. Man rührt 1 Stunde bei 0 °C und 3 Stunden bei Raumtemperatur, läßt über Nacht bei Raumtemperatur stehen, saugt anderntags den Niederschlag ab und engt das Filtrat ein. Der Rückstand wird zwischen Essigester und Wasser verteilt. Die Essigesterphase wird dann nacheinander mit gesättigter $NaHCO_3$-Lösung, $KHSO_4/K_2SO_4$-Puffer, gesättigter $NaHCO_3$-Lösung und Wasser ausgeschüttelt, über $Na_2SO_4$ getrocknet und eingeengt.
Ausbeute: 1,82 g ölige Substanz.

3 e. [5-(S)-(4-Amino-butyl)-2,4-dioxoimidazolidin-3-yl]-acetyl-Asp-Val-OH

1,82 g [5-(S)-(4-Tert.-butyloxycarboxamido-butyl)-2,4-dioxoimidazolidin-3-yl]-acetyl-Asp(OtBu)-Val-OtBu werden in 18 ml 90 %iger Trifluoressigsäure gelöst. Man läßt 1 Stunde bei Raumtemperatur stehen und engt im Vakuum ein. Der Rückstand wird in Wasser gelöst. Von Unlöslichem wird filtriert und das Filtrat gefriergetrocknet.
Ausbeute 1,8 g.

Zur Reinigung wird die Substanz an ®Sephadex LH20 in einer n-Butanol/Wasser/Essigsäure-Mischung chromatographiert. Die reinen Fraktionen werden vereinigt, eingeengt und der Rückstand in Wasser gelöst und gefriergetrocknet.
Ausbeute: 0,78 g,
$[\alpha]_D^{23}$ = - 58,8 ° (c = 1, in Wasser)

Beispiel 4:

[5-(S)-(3-Guanidinopropyl)-2,4-dioxoimidazolidin-3-yl]-acetyl-β-alanin

4 a. [5-(S)-(3-Guanidinopropyl)-2,4-dioxoimidazolidin-3-yl]-acetyl-β-alanin-tert.-butylester

Zu einer Suspension von 0,73 g HCl•H-β-Ala-OtBu, 1,03 g [5-(S)-(3-Guanidinopropyl)-2,4-dioxoimidazolidin-3-yl]-essigsäure und 0,54 g HOBt in 30 ml Dimethylformamid gibt man bei 0 °C 0,88 g DCC. Man rührt 1 Stunde bei 0 °C und 3 Stunden bei Raumtemperatur. Anschließend läßt man über Nacht bei Raumtemperatur stehen und saugt von Unlöslichem ab. Das Filtrat wird eingeengt und der Rückstand über ®Sephadex LH20 in einer Mischung aus Eisessig, Butanol und Wasser chromatographiert. Die Fraktionen mit der reinen Substanz werden eingeengt, in Wasser gelöst und gefriergetrocknet.
Ausbeute: 1,318 g amorphe Substanz.

4 b. [5-(S)-(3-Guanidinopropyl)-2,4-dioxoimidazolidin-3-yl]-acetyl-β-alanin

1,3 g [5-(S)-(3-Guanidinopropyl)-2,4-dioxoimidazolidin-3-yl]-acetyl-β-alanin-tert.-butylester werden in 15 ml 90 %iger wäßriger Trifluoressigsäure gelöst. Man läßt 1 Stunde bei Raumtemperatur stehen, und engt ein. Der Rückstand wird in Wasser gelöst und 3 mal mit Diethylether ausgeschüttelt. Die wäßrige Lösung wird von Unlöslichem filtriert und gefriergetrocknet.
Ausbeute 1,16 g,
$[\alpha]_D^{22}$ = 0,1 (c = 1, in Wasser).

Beispiel 5:

[5-(S,R)-(4-Formamidino-benzyl)-2,4-dioxoimidazolidin-3-yl]-acetyl-L-aspartyl-L-phenylglycin

5 a. [5-(S,R)-(4-Formamidino-benzyl)-2,4-dioxoimidazolidin-3-yl]-acetyl-Asp(OtBu)-L-phenylglycin-OtBu

Analog Beispiel 1d läßt man 538 mg [5-(S,R)-(4-Formamidino-benzyl)-2,4-dioxoimidazolidin-3-yl]-essigsäure und 714 mg HCl•H-Asp(OtBu)-L-phenylglycin-OtBu mit 232 mg HOBt und 375 mg DCC in 5 ml Dimethylformamid reagieren.
Ausbeute nach Reinigung: 1,01 g amorphe Substanz.

5 b. [5-(S,R)-(4-Formamidino-benzyl)-2,4-dioxoimidazoli-din-3-yl]-acetyl-L-aspartyl-L-phenylglycin

1,01 g [5-(S,R)-(4-Formamidino-benzyl)-2,4-dioxoimidazolidin-3-yl]-acetyl-Asp(OtBu)-L-phenylglycin-OtBu werden in 6 ml 90 %iger Trifluoressigsäure gelöst. Man läßt 1 Stunde bei Raumtemperatur stehen und engt ein. Der Rückstand wird in Wasser gelöst, von Unlöslichem filtriert und gefriergetrocknet.
Ausbeute: 832 mg,
$[\alpha]_D^{28}$ = + 9,7 ° (c = 1, in Wasser).

Beispiel 6:

[5-(S,R)-(4-Formamidino-benzyl)-2,4-dioxoimidazolidin-3-yl]-acetyl-β-alanin

6 a. [5-(S,R)-(4-Formamidino-benzyl)-2,4-dioxoimidazolidin-3-yl]-acetyl-β-alanin-OtBu

Analog Beispiel 1d läßt man 500 mg [5-(S,R)-(4-Formamidino-benzyl)-2,4-dioxoimidazolidin-3-yl]-essigsäure und 312 mg β-Alanin-OtBu•HCl mit 232 mg HOBt und 375 mg DCC reagieren.
Ausbeute nach Reinigung: 500 mg

6 b. [5-(S,R)-(4-Formamidino-benzyl)-2,4-dioxoimidazolidin-3-yl]-acetyl-β-alanin

500 mg [5-(S,R)-(4-Formamidino-benzyl)-2,4-dioxoimidazolidin-3-yl]-acetyl-β-alanin-OtBu werden in 5 ml 90 %iger Trifluoressigsäure gelöst. Man läßt 1 Stunde bei Raumtemperatur stehen und engt ein. Der Rückstand wird in Wasser gelöst, von Unlöslichem filtriert und das Filtrat gefriergetrocknet.
Ausbeute: 388 mg,
$[\alpha]_D^{28}$ = 0 ° (c = 1, in Wasser).

Beispiel 7:

(5-(S)-(4-Aminobenzyl)-2,4-dioxoimidazolidin-3-yl)-acetyl-L-aspartyl-L-valin

7 a. N-(1-Methoxycarbonyl-2(S)-(4-nitrophenyl)-ethyl)-N'-ethoxycarbonylmethyl- harnstoff

Zu 12,5 g (48 mmol) H-Phe-(4-NO2)-OMe•HCl und 6,2 g (48 mmol) Isocyanatoessigsäureethylester in 100 ml Dimethylformamid werden bei Raumtemperatur 6,1 ml (48 mmol) N-Ethylmorpholin zugetropft. Man rührt 4 Stunden, saugt das ausgefallene Produkt ab, fällt das Filtrat mit Wasser ab, saugt das weiter ausgefallene Produkt ab und trocknet über Phosphorpentoxid.
Ausbeute: 16,2 g (95 %)
Schmelzpunkt: 180 - 181 °C

7 b. (5-(S)-(4-Nitrobenzyl)-2,4-dioxoimidazolidin-3-yl)-essigsäure

3,45 g (9,8 mmol) N-(1-Methoxycarbonyl-2(S)-(4-nitrophenyl)-ethyl)-N'-ethoxycarbonylmethyl-harnstoff werden mit 40 ml 6N HCl und 20 ml Essigsäure 30 Minuten unter Rückfluß erhitzt. Das beim Abkühlen auskristallisierte Produkt wird abgesaugt und getrocknet.
Ausbeute: 2,5 g (87 %)
Schmelzpunkt: 211 - 213 °C

7 c. (5-(S)-(4-Nitrobenzyl)-2,4-dioxoimidazolidin-3-yl)-acetyl-Asp(OtBu)-Val-OtBu

Zu einer Suspension von 150 mg (0,5 mmol) (5-(S)(4-Nitrobenzyl)-2,4-dioxoimidazolidin-3-yl)-essigsäure, 190 mg (0,5 mmol) H-Asp-(OtBu)-Val-OtBu•HCl, 68 mg (0,5 mmol) HOBt und 71 µl (0,5 mmol) N-Ethylmorpholin in 5 ml Dimethylformamid gibt man bei 0 °C 115 mg (0,55 mmol) DCC. Man rührt 1 Stunde bei 0 °C und 4 Stunden bei Raumtemperatur, saugt den Niederschlag ab, engt das Filtrat ein, löst in Methylenchlorid und extrahiert mit Natriumhydrogencarbonatlösung und mit Kaliumhydrogensulfatlösung. Die organische Phase wird eingeengt.
Ausbeute: 300 mg (enthält noch etwas Dicyclohexylharnstoff)
Schmelzpunkt: 90 °C

7 d. (5-(S)-(4-Nitrobenzyl)-2,4-dioxoimidazolidin-3-yl)-acetyl-L-aspartyl-L-valin

270 mg (0,436 mmol) 5-(S)-(4-Nitrobenzyl)-2,4-dioxoimidazolidin-3-yl)-acetyl-Asp(OtBu)-Val-OtBu werden mit 4,5 ml Trifluoressigsäure und 0,5 ml Wasser versetzt. Nach einer Stunde wird eingeengt und gefriergetrocknet. Ausbeute: 220 mg.

7 e. (5-(S)-(4-Aminobenzyl)-2,4-dioxoimidazolidin-3-yl)-acetyl-L-aspartyl-L-valin

220 mg (0,43 mmol) (5-(S)-(4-Nitrobenzyl)-2,4-dioxoimidazolidin-3-yl)-acetyl-L-aspartyl-L-valin werden in 50 ml Methanol gelöst. Nach Zugabe von 10 mg 10 %-Pd auf Kohle wird 3 Stunden bei Raumtemperatur hydriert, vom Katalysator abfiltriert, eingeengt und gefriergetrocknet.
Ausbeute: 150 mg

Zur Abtrennung von Verunreinigungen wird über ®Sephadex LH20 in Butanol/Essigsäure/Wasser chromatographiert.
Ausbeute: 75 mg
Schmelzpunkt: 175 - 178 °C

Beispiel 8:

(5-(S)-(4-Guanidinobenzyl)-2,4-dioxoimidazolidin-3-yl)-acetyl-L-aspartyl-L-valin

8 a. N-(1-Methoxycarbonyl-2-(S)-(4-aminophenyl)ethyl)-N'-ethoxycarbonylmethyl- harnstoff

7,1 g (20 mmol) N-(1-Methoxycarbonyl-2-(S)-(4-nitrophenyl)-ethyl)-N'-ethoxycarbonylmethyl-harnstoff werden in 80 ml Dimethylformamid gelöst und nach Zugabe von 100 mg 10 %-Pd auf Kohle wird 5 Stunden bei Raumtemperatur hydriert, vom Katalysator abfiltriert und eingeengt.
Ausbeute: 7,7 g (enthält noch DMF)

8 b. (5-(S)-(4-Aminobenzyl)-2,4-dioxoimidazolidin-3-yl)-essigsäure-hydrochlorid

1 g (3,1 mmol) N-(1-Methoxycarbonyl-2-(S)-(4-aminophenyl)-ethyl)-N'-ethoxycarbonylmethyl-harnstoff werden in 10 ml 6N HCl 30 Minuten unter Rückfluß erhitzt. Nach dem Einengen erhält man 1 g eines hygroskopischen Harzes.

Alternativ kann die freie Base von 8b. wie folgt hergestellt werden:
1,1 g (3,75 mmol) (5-(S)-(4-Nitrobenzyl)-2,4-dioxoimidazolidin-3-yl)-essigsäure werden in 50 ml Methanol gelöst. Nach Zugabe von 50 mg 10 %-Pd auf Kohle wird 3 Stunden bei Raumtemperatur hydriert, vom Katalysator abfiltriert, eingeengt und gefriergetrocknet.
Ausbeute: 0,9 g (91 %)
Schmelzpunkt: 130 °C

8 c. (5-(S)-(4-Nitroguanidinobenzyl)-2,4-dioxoimidazolidin-3-yl)-essigsäure

680 mg (5 mmol) Nitro-S-methyl-isothioharnstoff und 900 mg (3,4 mmol) (5-(S)-(4-Aminobenzyl)-2,4-dioxoimidazolidin-3-yl)-essigsäure werden in 35 ml 0,1 N Natronlauge 5,5 Stunden bei 80 °C gerührt. Nach dem Abkühlen wird mit Methylenchlorid und Essigester extrahiert und die Wasserphase mit Salzsäure auf pH 2 - 3 angesäuert und eingeengt. Der Rückstand wird mit wenig Wasser verrührt und abgesaugt.
Ausbeute: 430 mg (36 %), weiteres Produkt ist aus dem Filtrat erhältlich

8 d. (5-(S)-(4-Nitroguanidinobenzyl)-2,4-dioxoimidazolidin-3-yl)-acetyl-Asp(OtB u)-Val-OtBu

Zu einer Suspension von 70 mg (0,2 mmol) (5-(S)-(4-Nitroguanidinobenzyl)-2,4-dioxoimidazolidin-3-y l)-essigsäure, 76 mg (0,2 mmol) H-Asp-(OtBu)-Val-OtBu • HCl, 27 mg (0,2 mmol) HOBt und 35 µl (0,27 mmol) N-Ethylmorpholin in 3 ml Dimethylformamid gibt man bei 0 °C 45 mg (0,22 mmol) DCC. Man rührt 1 Stunde bei 0 °C und 3 Stunden bei Raumtemperatur, saugt den Niederschlag ab, engt das Filtrat ein, löst in Methylenchlorid und extrahiert mit Natriumhydrogencarbonatlösung und mit Kaliumhydrogensulfatlösung. Die organische Phase wird eingeengt und gefriergetrocknet.
Ausbeute: 145 mg (enthält noch etwas Dicyclohexylharnstoff)

8 e. (5-(S)-(4-Nitroguanidinobenzyl)-2,4-dioxoimidazolidin-3-yl)-acetyl-L-aspartyl-L-valin

130 mg (5-(S)-(4-Nitroguanidinobenzyl)-2,4-dioxoimidazolidin-3-yl)-acetyl-Asp(OtBu)-Val-OtBu werden mit 4,5 ml Trifluoressigsäure und 0,5 ml Wasser versetzt. Nach einer Stunde wird eingeengt und gefriergetrocknet.
Ausbeute: 140 mg (enthält noch etwas Dicyclohexylharnstoff).

8 f. (5-(S)-(4-Guanidinobenzyl)-2,4-dioxoimidazolidin-3-yl)-acetyl-L-aspartyl-L-valin

120 mg (0,21 mmol) (5-(S)-(4-Nitroguanidinobenzyl)-2,4-dioxoimidazolidin-3-yl)-acetyl-L-aspartyl-L-valin werden in 50 ml Methanol gelöst. Nach Zugabe von 20 mg 10 %-Pd auf Kohle wird 4 Stunden bei Raumtemperatur hydriert, vom Katalysator abfiltriert, eingeengt und gefriergetrocknet.
Ausbeute: 50 mg (45 %), (hygroskopische Substanz)

Beispiel 9:

(5-(4-Aminomethylbenzyl)-1-methyl-2,4-dioxoimidazolidin-3-yl)-acetyl-L-aspartyl-L-valin

9 a. N-Methyl-N-ethoxycarbonylmethyl-N'-ethoxycarbonylmethyl-harnstoff

Zu 7,7 g (50 mmol) Sarcosinethylester•HCl und 6,5 g (50 mmol) Isocyanatoessigsäureethylester in 30 ml Dimethylformamid werden bei Raumtemperatur 6,4 ml (50 mmol) N-Ethylmorpholin zugetropft. Man rührt 4 Stunden, saugt das ausgefallene N-Ethylmorpholin•HCl ab und engt ein. Das erhaltene Öl kristallisiert beim Stehen, wird mit tert.-Butylmethylether verrührt und abgesaugt. Ausbeute: 11,9 g (97 %).
Schmelzpunkt: 82 - 85 °C

9 b. (1-Methyl-2,4-dioxoimidazolidin-3-yl)-essigsäure

10,5 g (42,6 mmol) N-Methyl-N-ethoxycarbonylmethyl-N-ethoxycarbonylmethyl-harnstoff werden in 150 ml 6N HCl 60 Minuten unter Rückfluß erhitzt. Nach dem Einengen und Gefriertrocknen erhält man 7,4 g Produkt.

9 c. (5-(4-Cyanobenzyliden)-1-methyl-2,4-dioxoimidazolidin-3-yl)-essigsäure

2,6 g (15 mmol) (1-Methyl-2,4-dioxoimidazolidin-3-yl)-essigsäure, 2,9 g (22 mmol) 4-Cyanbenzaldehyd, 1,8 g (22 mmol) Natriumacetat und 2,1 ml (22 mmol) Essigsäureanhydrid werden in 25 ml Essigsäure 6 Stunden unter Rückfluß erhitzt. Nach dem Abkühlen wird auf Eis gegossen und mit Essigester extrahiert. Die Essigesterphase wird mit Natriumhydrogencarbonatlösung extrahiert und die Wasserphase angesäuert. Das ausgefallene Produkt wird abgesaugt und getrocknet.
Ausbeute: 0,62 g
Schmelzpunkt: 240 - 245 °C
Weiteres Produkt kann aus dem Filtrat erhalten werden.

9 d. (5-(4-Cyanobenzyliden)-1-methyl-2,4-dioxoimidazolidin-3-yl)-acetyl-Asp(OtBu)-Val-OtBu

Zu 150 mg (0,53 mmol) (5-(4-Cyanobenzyliden)-1-methyl-2,4-dioxoimidazolidin-3-yl)-essigsäure, 200 mg (0,53 mmol) H-Asp-(OtBu)-Val-OtBu•HCl, 66 mg (0,49 mmol) H0Bt und 110 µl (0,86 mmol) N-Ethylmorpholin in 10 ml Dimethylformamid gibt man bei 0 °C 120 mg (0,58 mmol) DCC. Man rührt 1 Stunde bei 0 °C und 5,5 Stunden bei Raumtemperatur, saugt den Niederschlag ab, engt das Filtrat ein, löst in Methylenchlorid und extrahiert mit Natriumhydrogencarbonatlösung und mit Kaliumhydrogensulfatlösung. Die organische Phase wird eingeengt und gefriergetrocknet. Ausbeute: 360 mg (enthält noch etwas Dicyclohexylharnstoff)

9 e. (5-(4-Cyanobenzyliden)-1-methyl-2,4-dioxoimidazolidin-3-yl)-acetyl-L-aspartyl-L-valin

360 mg (5-(4-Cyanobenzyliden)-1-methyl-2,4-dioxoimidazolidin-3-yl)-acetyl-Asp(OtBu)-Val-OtBu werden mit 4,5 ml Trifluoressigsäure und 0,5 ml Wasser versetzt. Nach einer Stunde wird eingeengt und gefriergetrocknet. Ausbeute: 340 mg (enthält noch etwas Dicyclohexylharnstoff).

9 f. (5-(4-Aminomethylbenzyl)-1-methyl-2,4-dioxoimidazolidin-3-yl)-acetyl-L-aspartyl-L-valin

150 mg (5-(4-Cyanobenzyliden)-1-methyl-2,4-dioxoimidazolidin-3-yl)-acetyl-L-aspartyl-L-valin werden in 40 ml Methanol gelöst. Nach Zugabe von 50 mg 10 %-Pd auf Kohle wird 12 Stunden bei Raumtemperatur hydriert, vom Katalysator abfiltriert, eingeengt und gefriergetrocknet.

Beispiel 10

(5-(4-Aminomethylbenzyl)-2,4-dioxoimidazolidin-3-yl)-acetyl-L-aspartyl-L-valin

10 a.(5-(4-Cyanobenzyliden)-2,4-dioxoimidazolidin

36,7 g (0,28 mol) 4-Cyanbenzaldehyd, 10 g (0,1 mol) Hydantoin, 21,6 g (0,263 mol) Natriumacetat und 28,6 ml (0,3 mol) Essigsäureanhydrid werden in 85 ml Essigsäure 4 Stunden unter Rückfluß erhitzt. Nach dem Abkühlen wird auf Eis gegossen und mit Methylenchlorid extrahiert. Die Methylenchloridphase wird eingeengt, mit Methanol verrührt und abgesaugt.
Ausbeute: 9,9 g (47 %)
Schmelzpunkt: 310 - 315 °C

10 b. (5-(4-Cyanobenzyliden)-2,4-dioxoimidazolidin-3-yl)-essigsäurebenzylester

6,4 g (0,03 mol) (5-(4-Cyanobenzyliden)-2,4-dioxoimidazolidin und 3,5 g (0,031 mol) Kalium-tert.-butylat werden in 30 ml Dimethylformamid gelöst. Nach Zugabe von 7,1 g (0,031 mol) Bromessigsäurebenzylester wird 7 Stunden bei Raumtemperatur gerührt, kurz auf 100 °C erwärmt und eingeengt. Der Rückstand wird mit Wasser versetzt und mit Essigester extrahiert. Aus der Essigesterphase kristallisiert das Produkt aus oder kann durch Zugabe von Heptan ausgefällt werden.
Ausbeute: 7,5 g (69 %)

10 c. (5-(4-Cyanobenzyliden)-2,4-dioxoimidazolidin-3-yl)-essigsäure

0,4 g (1,1 mmol) (5-(4-Cyanobenzyliden)-2,4-dioxoimidazolidin-3-yl)-essigsäurebenzylester werden mit 5 ml 6N HCl und 5 ml Essigsäure 30 Minuten unter Rückfluß erhitzt, heiß filtriert und abgekühlt. Das ausgefallene Produkt wird abgesaugt, mit Wasser gewaschen und getrocknet.
Ausbeute: 150 mg (50 %)
Alternativ kann diese Verbindung wie folgt hergestellt werden:
5 g (32 mmol) Hydantoin-3-essigsäure, 6,3 g (48 mmol) 4-Cyanbenzaldehyd, 15 g (183 mmol) Natriumacetat und 10 ml (106 mmol) Essigsäureanhydrid werden in 30 ml Essigsäure 1,5 Stunden unter Rückfluß erhitzt. Nach dem Abkühlen wird auf Eis gegossen, mit konz. HCl auf pH 3 angesäuert, abgesaugt und aus Essigsäure umkristallisiert.
Ausbeute: 2 g
Weiteres Produkt kann aus dem Filtrat erhalten werden.

10 d. (5-(4-Cyanobenzyliden)-2,4-dioxoimidazolidin-3-yl)-acetyl-Asp(OtBu)-Val-OtBu

Zu 244 mg (0,9 mmol) (5-(4-Cyanobenzyliden)-2,4-dioxoimidazolidin-3-yl)-essigsäure, 350 mg (0,9 mmol) H-Asp-(OtBu)-Val-OtBu•HCl, 122 mg (0,9 mmol) HOBt und 104 mg (0,9 mmol) N-Ethylmorpholin in 50 ml Dimethylformamid gibt man bei 0 °C 210 mg (1 mmol) DCC. Man rührt 1 Stunde bei 0 °C und 5 Stunden bei Raumtemperatur, saugt den Niederschlag ab, engt das Filtrat ein, löst in Methylenchlorid und extrahiert mit Natriumhydrogencarbonatlösung und mit Kaliumhydrogensulfatlösung. Die organische Phase wird eingeengt und gefriergetrocknet.
Ausbeute: 640 mg (enthält noch etwas Dicyclohexylharnstoff)

10 e. (5-(4-Cyanobenzyliden)-2,4-dioxoimidazolidin-3-yl)-acetyl-L-aspartyl-L-valin

570 mg (0,95 mmol) (5-(4-Cyanobenzyliden)-2,4-dioxoimidazolidin-3-yl)-acetyl-Asp(OtBu)-Val-OtBu werden mit 5,4 ml Trifluoressigsäure und 0,6 ml Wasser 1 Stunde bei Raumtemperatur stehen gelassen und eingeengt. Ausbeute: 500 mg (enthält noch etwas Dicyclohexylharnstoff)

10 f. (5-(4-Aminomethylbenzyl)-2,4-dioxoimidazolidin-3-yl)-acetyl-L-aspartyl-L-valin

400 mg (5-(4-Cyanobenzyliden)-2,4-dioxoimidazolidin-3-yl)-acetyl-L-aspartyl-L-valin werden in 60 ml Methanol gelöst. Nach Zugabe von 50 mg 10 %-Pd auf Kohle wird 12 Stunden bei Raumtemperatur hydriert, vom Katalysator abfiltriert, eingeengt und gefriergetrocknet.

Beispiel 11:

3-(5-(S)-(3-Guanidinopropyl)-2,4-dioxoimidazolidin-3-yl)-benzoyl-L-aspartyl-L-valin

11 a. N-(1-Methoxycarbonyl-(4-(S)-nitroguanidino)-butyl)-N`-( 3-ethoxycarbonyl-phenyl-harnstoff

5 g (26 mmol) 3-Isocyanatobenzoesäureethylester und 7 g (26 mmol) H-Arg-(NO2)-OMe•HCl werden in 50 ml Dimethylformamid gelöst. Man tropft bei Raumtemperatur 5 ml (48 mmol) N-Ethylmorpholin zu, rührt 8 Stunden bei 50 °C, engt ein, löst in Methylenchlorid und extrahiert mit verdünnter Salzsäure. Die organische Phase wird eingeengt und über eine Kieselgelsäule in Methylenchlorid/ Methanol = 98 : 2 bis 90 : 10 chromatographiert. Ausbeute: 4,1 g (37 %) und 3,1 g der entsprechenden Säure

11 b. 3-(5-(S)-(3-Nitroguanidinopropyl)-2,4-dioxoimidazolidin-3-yl)-benzoesäure

2 g (4,7 mmol) N-(1-Methoxycarbonyl-(4-(S)-nitroguanidino)-butyl)-N'-(3-ethoxycarbonylphenyl)-harnstoff werden mit 40 ml 6N HCl 30 Minuten unter Rückfluß gekocht. Nach dem Abkühlen werden die ausgefallenen Kristalle abgesaugt und getrocknet.
Ausbeute: 1,8 g (95 %)
Schmelzpunkt: 105 -110 °C

11 c. 3-(5-(S)-(3-Nitroguanidinopropyl)-2,4-dioxoimidazolidin-3-yl)-benzoyl-Asp(OtBu)-Val-OtBu

Zu einer Suspension von 335 mg (0,92 mmol) 3-(5-(S)-(3-Nitroguanidino-propyl)-2,4-dioxoimidazolidin-3-yl)benzoesäure, 350 mg (0,92 mmol) H-Asp-(OtBu)-Val-OtBu•HCl, 130 mg (0,92 mmol) HOBt und 110 mg (0,92 mmol) N-Ethylmorpholin in 10 ml Dimethylformamid gibt man bei 0 °C 210 mg (1,01 mmol) DCC. Man rührt 1 Stunde bei 0 °C und 4 Stunden bei Raumtemperatur, saugt den Niederschlag ab, engt das Filtrat ein, löst in Methylenchlorid und extrahiert mit Natriumhydrogencarbonatlösung und mit Kaliumhydrogensulfatlösung. Die organische Phase wird eingeengt.
Ausbeute: 400 mg (63 %)

11 d. 3-(5-(S)-(3-Nitroguanidinopropyl)-2,4-dioxoimidazolidin-3-yl)-benzoyl-L-aspartyl-L-valin

400 mg (0,58 mmol) 3-(5-(S)-(3-Nitroguanidinopropyl)-2,4-dioxoimidazolidin-3-yl)-benzoyl-Asp(OtBu)-Val-OtBu werden mit 4,5 ml Trifluoressigsäure und 0,5 ml Wasser 1 Stunde bei Raumtemperatur stehen gelassen und eingeengt.
Ausbeute: 290 mg (94 %).

11 e. 3-(5-(S)-(3-Guanidinopropyl)-2,4-dioxoimidazolidin-3-yl)-benzoyl-L-aspartyl-L-valin

250 mg (0,36 mmol) 3-(5-(S)-(3-Nitroguanidinopropyl)-2,4-dioxoimidazolidin-3-yl)-benzoyl-L-aspartyl-L-valin werden in 30 ml Methanol gelöst. Nach Zugabe von 20 mg 10 %-Pd auf Kohle wird 4 Stunden bei Raumtemperatur hydriert, vom Katalysator abfiltriert, eingeengt und gefriergetrocknet.
$[\alpha]_D^{25}$ = -20,8 ° (c = 0,53, Wasser)

Beispiel 12:

(5-(R,S)-(4-Formamidinobenzyl)-2,4-dioxo-imidazolidin-3-yl)-acetyl-L-aspartyl-L-isoleucin

Diese Verbindung wurde analog der in Beispiel 1 beschriebenen Methode hergestellt.
$[\alpha]_D^{23}$ = - 31,6 ° (c = 1, Wasser)

Beispiel 13:

(5-(R,S)-(4-Formamidinobenzyl)-2,4-dioxo-imidazolidin-3-yl)-acetyl-L-aspartyl-L-lysin

Diese Verbindung wurde analog der in Beispiel 1 beschriebenen Methode hergestellt.
$[\alpha]_D^{23}$ = - 17,4 ° (c = 1, Wasser)

Beispiel 14:

(5-(R,S)-(4-Formamidinobenzyl)-2,4-dioxo-imidazolidin-3-yl)-acetyl-L-aspartyl-L-phenylalanin

Diese Verbindung wurde analog der in Beispiel 1 beschriebenen Methode hergestellt.
$[\alpha]_D^{23}$ = - 18,9 ° (c = 1, Wasser)

Beispiel 15:

(5-(4-Aminomethylbenzyliden)-2,4-dioxo-imidazolidin-3-yl)-acetyl-L-aspartyl-L-phenylglycin

Zu 90 mg (0,24 mmol) (5-(4-Tert.-Butyloxycarbonylaminomethylbenzyliden-2,4-dioxo-imidazolidin-3-yl)-essigsäure, 103 mg (0,24 mmol) H-Asp(OtBu)-phenylglycin-OtBu-hydrochlorid, 32 mg (0,24 mmol) Hydroxybenzotriazol in 15 ml Dimethylformamid gibt man bei 0 °C 28 mg (0,24 mmol) N-Ethylmorpholin und 54 mg (0,26 mmol) DCC. Man rührt 1 Stunde bei 0 °C und anschließend über Nacht bei Raumtemperatur. Das Reaktionsgemisch wird im Vakuum eingeengt, der Rückstand in Essigsäureethylester aufgenommen und die organische Phase mit Natriumhydrogencarbonatlösung, Kaliumhydrogencarbonatlösung und Wasser extrahiert, über Magnesiumsulfat getrocknet und eingeengt. Das erhaltene Produkt (200 mg) wird mit 5 ml 95 %iger Trifluoressigsäure 1 Stunde bei Raumtemperatur gerührt und im Vakuum eingeengt. Das Rohprodukt wird zur Reinigung an Sephadex LH20 mit einer homogenen Mischung von Butanol/Eisessig/Wasser chromatographiert.
Ausbeute: 100 mg
Schmelzpunkt: 48 °C

Beispiel 16:

(5-(R,S)-(4-Formamidinobenzyl)-2,4-dioxo-imidazolidin-3-yl)-acetyl-L-aspartyl-L-threonin

Diese Verbindung wurde analog der in Beispiel 1 beschriebenen Methode hergestellt.
$[\alpha]_D^{23}$ = - 20,1 ° (c = 1, Wasser)

Beispiel 17:

(5-(R,S)-(4-Formamidinobenzyl)-2,4-dioxo-imidazolidin-3-yl)-acetyl-L-aspartyl-L-phenylglycin-(1-hexadecylester)

Diese Verbindung wurde analog der in Beispiel 1 beschriebenen Methode hergestellt.
FAB-MS 763,5 $(M+H)^+$

Beispiel 18:

(5-(S)-(4-Guanidinobenzyl)-2,4-dioxo-imidazolidin-3-yl)-acetyl-L-aspartyl-L-phenylglycin

Diese Verbindung wurde analog der in Beispiel 1 beschriebenen Methode hergestellt.
Schmelzpunkt: 48°C
$[\alpha]_D^{20}$ = + 14 ° (c = 0,5, Wasser)

Beispiel 19:

(5-(4-Aminomethylbenzyl)-2,4-dioxo-imidazolidin-3-yl)-acetyl-L-aspartyl-L-phenylglycin

53 mg (0,1 mmol) (5-(4-Aminomethylbenzyliden)-2,4-dioxo-imidazolidin-3-yl)-acetyl-L-aspartyl-L-phenylglycin werden in 20 ml Methanol und 5 ml Dimethylformamid gelöst und nach Zugabe von 19 mg 10 %-Palladium auf Kohle bei Raumtemperatur hydriert. Der Katalysator wird abfiltriert, das Filtrat eingeengt und der Rückstand gefriergetrocknet.
FAB-MS 526,2 $(M+H)^+$

Beispiel 20:

(5-(R,S)-(4-Formamidinobenzyl)-2,4-dioxo-imidazolidin-3-yl)-acetyl-L-aspartyl-(N-methyl)-L-phenylglycin

Diese Verbindung wurde analog der in Beispiel 1 beschriebenen Methode hergestellt.
$[\alpha]_D^{23}$ = - 26,3 ° (c = 1, Wasser)

Beispiel 21:

(5-(R,S)-(4-Formamidinobenzyl)-2,4-dioxo-imidazolidin-3-yl)-acetyl-L-aspartyl-L-valinbenzylester

Diese Verbindung wurde analog der in Beispiel 1 beschriebenen Methode hergestellt.
$[\alpha]_D^{23}$ = - 37 ° (c = 1, Methanol)

Beispiel 22:

(5-(R,S)-(4-Formamidinobenzyl)-2,4-dioxo-imidazolidin-3-yl)-acetyl-L-aspartyl-L-tryptophan

Diese Verbindung wurde analog der in Beispiel 1 beschriebenen Methode hergestellt.
$[\alpha]_D^{24}$ = - 7,9 ° (c = 1, 80 %ige Essigsäure)

Beispiel 23:

(5-(S)-(4-Aminobutyl)-2,4-dioxo-imidazolidin-3-yl)-acetyl-L-aspartyl-L-valin

Diese Verbindung wurde analog der in Beispiel 1 beschriebenen Methode hergestellt.
$[\alpha]_D^{24}$ = - 58,8 ° (c = 1, Wasser)

Beispiel 24:

(5-(4-Aminobenzyliden)-2,4-dioxo-imidazolidin-3-yl)-acetyl-L-aspartyl-L-valin

Diese Verbindung wurde analog der in Beispiel 15 beschriebenen Methode hergestellt.
Schmelzpunkt: 160 °C
$[\alpha]_D^{25}$ = - 39,3 ° (c = 0,28, Wasser: Essigsäure = 95 : 5)

Beispiel 25:

(5-(4-Formamidinobenzyliden)-2,4-dioxo-imidazolidin-3-yl)-acetyl-L-aspartyl-L-phenylglycin

Diese Verbindung wurde analog der in Beispiel 15 beschriebenen Methode hergestellt.

Beispiel 26:

(5-(R,S)-(4-Formamidinobenzyl)-2,4-dioxo-imidazolidin-3-yl)-acetyl-L-aspartyl-L-phenylglycin

Diese Verbindung wurde analog der in Beispiel 1 beschriebenen Methode hergestellt.

Beispiel 27:

(5-(4-Formamidinobenzyliden)-1-methyl-2,4-dioxo-imidazolidin-3-yl)-acetyl-L-aspartyl-L-phenylglycin

Diese Verbindung wurde analog der in Beispiel 15 beschriebenen Methode hergestellt.

Beispiel 28:

(5-(R,S)-(4-Formamidinobenzyl)-1-methyl-2,4-dioxo-imidazolidin-3-yl)-acetyl-L-aspartyl-L-phenylglycin

Diese Verbindung wurde analog der in Beispiel 1 beschriebenen Methode hergestellt.

Beispiel 29:

(5-(3-Aminomethylbenzyl)-2,4-dioxo-imidazolidin-3-yl)-acetyl-L-aspartyl-L-phenylglycin

Diese Verbindung wurde analog der in Beispiel 15 beschriebenen Methode hergestellt.

Beispiel 30:

3-(5-(S)-(3-Aminopropyl)-2,4-dioxo-imidazolidin-3 -yl)-benzoyl-L-aspartyl-L-phenylglycin

Diese Verbindung wurde analog der in Beispiel 1 beschriebenen Methode hergestellt.

Beispiel 31:

(5-(R,S)-(4-Formamidinobenzyl)-2,4-dioxo-imidazolidin-3-yl)-acetyl-L-aspartyl-L-(1-hexadecanoyl)-lysin

Diese Verbindung wurde analog der in Beispiel 1 beschriebenen Methode hergestellt.

Beispiel 32:

(5-(S)-(4-Aminobutyl)-2,4-dioxo-imidazolidin-3-yl)-propionyl-L-aspartyl-L-valin

Diese Verbindung wurde analog der in Beispiel 1 beschriebenen Methode hergestellt.
$[\alpha]_D^{23}$ = - 47,5 ° (c = 1, Wasser)

Beispiel 33:

(5-(S)-(4-Aminobutyl)-2,4-dioxo-imidazolidin-3-yl)-propionyl-L-aspartyl-L-phenylglycin

Diese Verbindung wurde analog der in Beispiel 1 beschriebenen Methode hergestellt.
$[\alpha]_D^{23}$ = + 10,2 ° (c = 1, Wasser)

Pharmakologische Daten:

A) Geprüft wird die Hemmung der Bindung von Fibrinogen an seinen Rezeptor (Glykoprotein IIb/IIIa) an intakten, gelfiltrierten Human-Thrombozyten durch die erfindungsgemäßen Verbindungen. Angegeben ist der $K_i$-Wert der Bindungshemmung von $^{125}$I-Fibrinogen nach Stimulierung mit ADP (10 µM).

Literatur: J. S. Bennett u. G. Vilaire, J. Clin. Invest. 64 (1979), 1393 - 1401 E. Kornecki et al., J. Biol. Chem. 256 (1981), 5695 - 5701 G. A. Marguerie et al., J. Biol. Chem. 254 (1979), 5357 - 5363 G. A. Marguerie et al., J. Biol. Chem. 255 (1980), 154 - 161

| Beispiel | $K_i$ (µM), ADP stimuliert |
|---|---|
| 1 | 0,031 |
| 2 | 0,4 |
| 5 | 0,022 |
| 6 | 1,91 |
| 7 | 2,43 |
| 8 | 0,71 |
| 11 | 1,87 |
| 12 | 0,042 |
| 13 | 0,042 |
| 14 | 0,27 |
| 15 | 0,28 |
| 16 | 0,12 |
| 20 | 1,59 |
| 21 | 0,24 |
| 22 | 0,14 |
| 32 | 2,71 |

B) Als funktioneller Test wird die Hemmung der Aggregation gelfiltrierter Human-Thrombozyten nach ADP-oder Thrombin-Stimulierung durch die erfindungsgemäßen Verbindungen gemessen. Angegeben ist der $IC_{50}$-Wert der Hemmung.

Literatur: G. A. Marguerie et al., J. Biol. Chem. 254 (1979), 5357 - 5363

| Beispiel | $IC_{50}$ (µM), | |
|---|---|---|
| | ADP-stimuliert | Thrombin-stimuliert |
| 1 | 0,4 | 0,15 |
| 2 | 4,5 | 1,5 |
| 3 | 30 | 4 |
| 5 | 0,2 | 0,1 |
| 6 | 5,5 | 3 |
| 8 | 6 | 3 |
| 12 | 0,2 | 0,1 |
| 13 | 0,35 | 0,4 |
| 14 | 0,55 | 0,3 |
| 15 | 0,7 | 0,45 |
| 16 | 0,7 | 0,2 |
| 17 | 4,5 | 4 |
| 18 | 5,5 | 2 |
| 19 | 7 | 2,5 |
| 20 | 4,5 | 2,5 |
| 21 | 0,7 | 0,3 |
| 22 | 0,8 | 0,25 |
| 32 | 6 | 2 |

## Patentansprüche

**Patentansprüche für folgende Vertragsstaaten : AT, BE, CH, DE, DK, FR, GB, IT, LU, NL, SE**

**1.** Verbindung der Formel I,

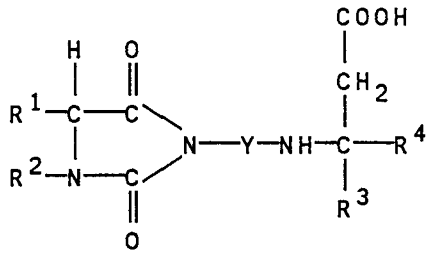

(I)

in welcher
Y $-(CH_2)_m-CO-$ oder

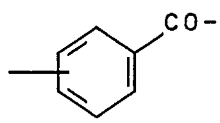

bedeutet und m für 1 - 4 steht;
$R^1$ für $-(CH_2)_n$-NH-X, $-CH_2-C_6H_4$-NH-X, $-CH_2-C_6H_4-C(=NH)-NH_2$, $-CH_2-C_6H_4-CH_2$-NH-X oder $-C_6H_4$-NH-X steht oder

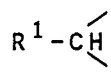

auch $>C=CH-C_6H_4-X^1$ bedeutet,
wobei n für eine ganze Zahl 3 - 5 steht,
$X^1$ $-CH_2NHX$, -NHX oder $-C(=NH)-NH_2$ bedeutet,
X für Wasserstoff, $C_1$-$C_6$-Alkyl oder für einen Rest der Formel II steht,

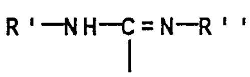

(II)

worin
R' und R'' unabhängig voneinander Wasserstoff oder $C_1$-$C_6$-Alkyl bedeuten;
$R^2$ Wasserstoff oder $C_1$-$C_6$-Alkyl bedeutet;
$R^3$ Wasserstoff oder einen Phenylrest bedeutet;
$R^4$ Wasserstoff, $COOR^5$, $CO-N(CH_3)-R^5$ oder $CO-NH-R^5$ bedeutet, worin
$R^5$ Wasserstoff oder unsubstituiertes oder ein- oder mehrfach durch gleiche oder verschiedene Reste $R^6$ substituiertes $C_1$-$C_8$-Alkyl bedeutet;
$R^6$ Hydroxy, Carboxy, Carboxamido, Amino, Mercapto, $C_1$-$C_8$-Alkoxy, $C_1$-$C_{18}$-Alkoxycarbonyl, Phenyl-$C_1$-$C_3$-alkoxycarbonyl, $C_3$-$C_8$-Cycloalkyl, Halogen, Nitro, Trifluormethyl, Phenyl, Phenyl-$C_1$-$C_8$-alkyl, $-NR^7R^8$, $-OR^7$, $-SR^7$, $-CONR^7R^8$, $-COOR^7$, $-COSR^7$, eine Aminosäureseitenkette, einen natürlichen oder unnatürlichen Aminosäure-, Iminosäure- oder einen Dipeptid-Rest sowie deren Ester und Amide bedeutet, wobei freie funktionelle Gruppen gegebenenfalls durch in der Peptidchemie üblichen Schutzgruppen geschützt sein können;
$R^7$ Wasserstoff, gegebenenfalls durch eine Aminogruppe substituiertes Phenyl-$C_1$-$C_8$-alkyl, $C_1$-$C_{18}$-Alkylcarbonyl, $C_1$-$C_8$-Alkyloxycarbonyl, Phenylcarbonyl, Phenyl-$C_1$-$C_8$-alkylcarbonyl, Phenyl-$C_1$-$C_8$-al-

kyloxycarbonyl, einen natürlichen oder unnatürlichen Aminosäure-, Iminosäure- oder einen Dipeptid-Rest bedeutet;
$R^8$ Wasserstoff, $C_1$-$C_8$-Alkyl, Phenyl oder Phenyl-$C_1$-$C_8$-alkyl bedeutet;
sowie deren physiologisch verträgliche Salze, wobei Verbindungen der Formel I ausgenommen sind, worin
$R^1$ -$(CH_2)_{3-4}$ - NH - X mit
X = $C_1$-$C_6$-Alkyl oder einen Rest der Formel II;
$R^2$ und $R^3$ Wasserstoff;
$R^4$ -CO-NH-$R^5$ und
Y -$CH_2$-CO bedeuten.

2. Verbindung der Formel I gemäß Anspruch 1, dadurch gekennzeichnet, daß
$R^1$ -$CH_2$-$C_6H_4$-C(NH)-$NH_2$ oder -$CH_2$-$C_6H_4$-$CH_2$-$NH_2$,
$R^2$ H oder $CH_3$,
Y -$CH_2$-CO- und
$R^4$ -CO-NH-$R^5$ bedeuten, wobei NH-$R^5$ für einen α-Aminosäurerest, bevorzugt einen Valin- oder Phenylglycin-Rest, steht.

3. Verfahren zur Herstellung einer Verbindung der Formel I gemäß Anspruch 1 oder 2 durch Fragmentkondensation von z. B. Verbindungen der Formel IIIa, IIIb oder IIIc mit Verbindungen der Formel IV, wobei $R^1$, $R^2$, $R^3$, $R^4$, $X^1$ und m die oben genannten Bedeutungen haben:

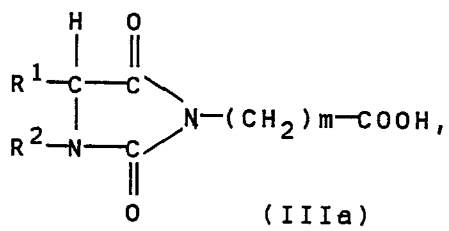
(IIIa)

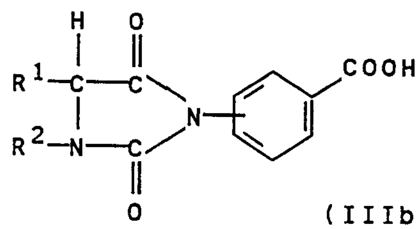
(IIIb)

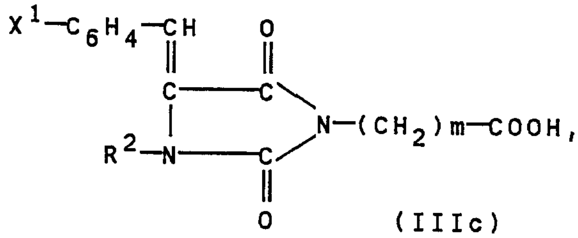
(IIIc)

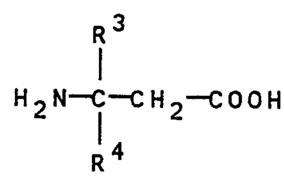
(IV)

4. Verbindung der Formel I gemäß den Ansprüchen 1 oder 2 zur Anwendung als Heilmittel.

5. Verwendung einer Verbindung der Formel I gemäß den Ansprüchen 1 oder 2 zur Herstellung von Arzneimitteln zur Hemmung der Thrombozytenaggregation.

6. Verwendung einer Verbindung der Formel I gemäß den Ansprüchen 1 oder 2 zur Herstellung von Arzneimitteln zur Hemmung der Osteoclastenbindung an die Knochenoberfläche.

7. Pharmazeutische Zubereitung enthaltend eine Verbindung der Formel I gemäß den Ansprüchen 1 oder 2 oder deren physiologisch verträgliches Salz und einen annehmbaren Träger.

**Patentansprüche für folgende Vertragsstaaten : ES, GR**

**1.** Verfahren zur Herstellung einer Verbindung der Formel I,

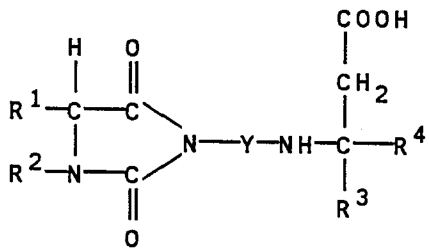

(I)

in welcher
Y $-(CH_2)_m-CO-$ oder

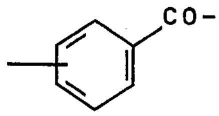

bedeutet und m für 1 - 4 steht;
$R^1$ für $-(CH_2)_n$-NH-X, $-CH_2-C_6H_4$-NH-X, $-CH_2-C_6H_4-C(=NH)-NH_2$, $-CH_2-C_6H_4-CH_2$-NH-X oder $-C_6H_4$-NH-X steht oder

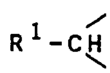

auch $>C=CH-C_6H_4-X^1$
bedeutet,
wobei n für eine ganze Zahl 3 - 5 steht,
$X^1$ $-CH_2NHX$, -NHX oder $-C(=NH)-NH_2$ bedeutet,
X für Wasserstoff, $C_1$-$C_6$-Alkyl oder für einen Rest der Formel II steht,

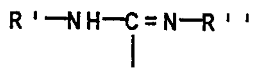

(II)

worin
R' und R'' unabhängig voneinander Wasserstoff oder $C_1$-$C_6$-Alkyl bedeuten;
$R^2$ Wasserstoff oder $C_1$-$C_6$-Alkyl bedeutet;
$R^3$ Wasserstoff oder einen Phenylrest bedeutet;
$R^4$ Wasserstoff, $COOR^5$, $CO-N(CH_3)-R^5$ oder $CO-NH-R^5$ bedeutet, worin
$R^5$ Wasserstoff oder unsubstituiertes oder ein- oder mehrfach durch gleiche oder verschiedene Reste $R^6$ substituiertes $C_1$-$C_8$-Alkyl bedeutet;
$R^6$ Hydroxy, Carboxy, Carboxamido, Amino, Mercapto, $C_1$-$C_8$-Alkoxy, $C_1$-$C_{18}$-Alkoxycarbonyl, Phenyl-$C_1$-$C_3$-alkoxycarbonyl, $C_3$-$C_8$-Cycloalkyl, Halogen, Nitro, Trifluormethyl, Phenyl, Phenyl-$C_1$-$C_8$-alkyl, $-NR^7R^8$, $-OR^7$, $-SR^7$, $-CONR^7R^8$, $-COOR^7$, $-COSR^7$, eine Aminosäureseitenkette, einen natürlichen oder unnatürlichen Aminosäure-, Iminosäure- oder einen Dipeptid-Rest sowie deren Ester und Amide bedeutet, wobei freie funktionelle Gruppen gegebenenfalls durch in der Peptidchemie üblichen Schutzgruppen geschützt sein können;
$R^7$ Wasserstoff, gegebenenfalls durch eine Aminogruppe substituiertes Phenyl-$C_1$-$C_8$-alkyl, $C_1$-$C_{18}$-Alkylcarbonyl, $C_1$-$C_8$-Alkyloxycarbonyl, Phenylcarbonyl, Phenyl-$C_1$-$C_8$-alkylcarbonyl, Phenyl-$C_1$-$C_8$-alkyloxycarbonyl, einen natürlichen oder unnatürlichen Aminosäure-, Iminosäure- oder einen Dipeptid-Rest bedeutet;

$R^8$ Wasserstoff, $C_1$-$C_8$-Alkyl, Phenyl oder Phenyl-$C_1$-$C_8$-alkyl bedeutet;
sowie deren physiologisch verträgliche Salze, wobei Verbindungen der Formel I ausgenommen sind, worin
$R^1$ -$(CH_2)_{3-4}$ - NH - X mit
X = $C_1$-$C_6$-Alkyl oder einen Rest der Formel II;
$R^2$ und $R^3$ Wasserstoff;
$R^4$ -CO-NH-$R^5$ und
Y -$CH_2$-CO bedeuten,
durch Fragmentkondensation von z. B. Verbindungen der Formel IIIa, IIIb oder IIIc mit Verbindungen der Formel IV, wobei $R^1$, $R^2$, $R^3$, $R^4$, $X^1$ und m die oben genannten Bedeutungen haben:

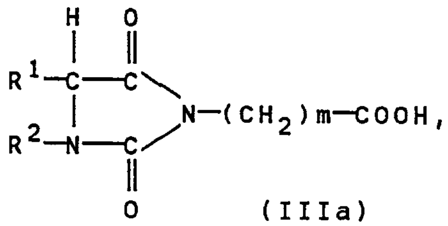

(IIIa)

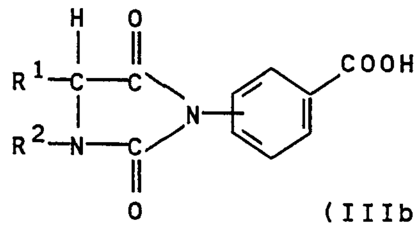

(IIIb)

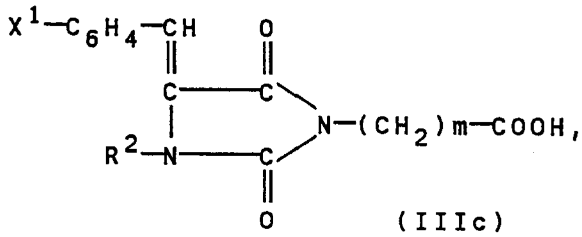

(IIIc)

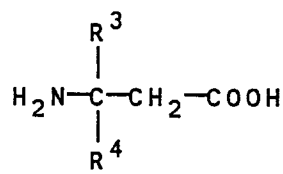

(IV)

2. Verfahren gemäß Anspruch 1, dadurch gekennzeichnet, daß in der Formel I
$R^1$ -$CH_2$-$C_6H_4$-C(NH)-$NH_2$ oder -$CH_2$-$C_6H_4$-$CH_2$-$NH_2$,
$R^2$ H oder $CH_3$,
Y -$CH_2$-CO- und
$R^4$ -CO-NH-$R^5$ bedeuten, wobei NH-$R^5$ für einen $\alpha$-Aminosäurerest, bevorzugt einen Valin- oder Phenylglycin-Rest, steht.

3. Verwendung einer gemäß Anspruch 1 und/oder 2 hergestellten Verbindung der allgemeinen Formel I zur Herstellung von Arzneimitteln zur Hemmung der Thrombozytenaggregation.

4. Verwendung einer gemäß Anspruch 1 und/oder 2 hergestellten Verbindung der allgemeinen Formel I zur Herstellung von Arzneimitteln zur Hemmung der Osteoclastenbindung an die Knochenoberfläche.

5. Verfahren zur Herstellung einer pharmazeutischen Zubereitung, enthaltend eine nach einem der Verfahren der Ansprüche 1 und/oder 2 hergestellten Verbindung der Formel I, dadurch gekennzeichnet, daß man diese zusammen mit einem annehmbaren Träger in eine geeignete Darreichungsform bringt.

## Claims

### Claims for the following Contracting States : AT, BE, CH, DE, DK, FR, GB, IT, LU, NL, SE

**1.** Compound of the formula I,

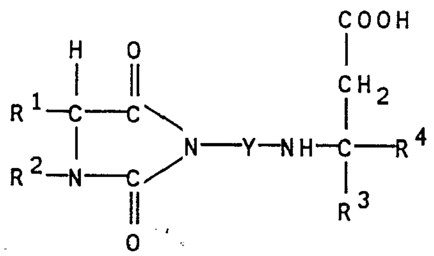

(I)

in which
Y denotes $-(CH_2)_m-CO-$ or

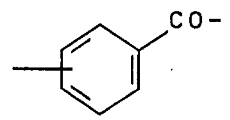

and m represents 1 - 4;
$R^1$ represents $-(CH_2)n-NH-X$, $-CH_2-C_6H_4-NH-X$, $-CH_2-C_6H_4-C(=NH)-NH_2$, $-CH_2-C_6H_4-CH_2-NH-X$ oder $-C_6H_4-NH-X$ or

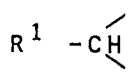

also denotes $>C=CH-C_6H_4-X^1$,
wherein n represents an integer from 3 to 5,
$X^1$ denotes $-CH_2NHX$, $-NHX$ oder $-C(=NH)-NH_2$,
x represents hydrogen or $C_1$-$C_6$-alkyl, or represents a radical of the formula II,

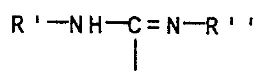

(II)

wherein
R' und R"independently of one another denote hydrogen or $C_1$-$C_6$-alkyl;
$R^2$ denotes hydrogen or $C_1$-$C_6$-alkyl;
$R^3$ denotes hydrogen or a phenyl radical;
$R^4$ denotes hydrogen, $COOR^5$, $CO-N(CH_3)-R^5$ or $CO-NH-R^5$, wherein
$R^5$ denotes hydrogen or $C_1$-$C_8$-alkyl, which is unsubstituted or mono- or polysubstituted by identical or different radicals $R^6$;
$R^6$ denotes hydroxy, carboxy, carboxamido, amino, mercapto, $C_1$-$C_8$-alkoxy, $C_1$-$C_{18}$-alkoxycarbonyl, phenyl-$C_1$-$C_3$-alkoxycarbonyl, $C_3$-$C_8$-cycloalkyl, halogen, nitro and trifluoromethyl, phenyl, phenyl-$C_1$-$C_8$-alkyl, $-NR^7R^8$, $-OR^7$, $-SR^7$, $-CONR^7R^8$, $-COOR^7$, $-COSR^7$, an amino acid side chain, a naturally occurring or unnatural amino acid radical, imino acid radical or a dipeptide radical as well as esters or amides thereof, it being possible for free functional groups optionally to be protected by protective groups which are customary in peptide chemistry;
$R^7$ denotes hydrogen, phenyl-$C_1$-$C_8$-alkyl, $C_1$-$C_{18}$-alkylcarbonyl, $C_1$-$C_8$-alkyloxycarbonyl, phenylcarbonyl, phenyl-$C_1$-$C_8$-alkylcarbonyl, phenyl-$C_1$-$C_8$-alkyloxycarbonyl which may be substituted by an amino group, a naturally occurring or unnatural amino acid radical, imino acid radical or a dipeptide

radical;
$R^8$ denotes hydrogen, $C_1$-$C_8$-alkyl, phenyl or phenyl -$C_1$-$C_8$-alkyl and physiologically tolerated salts thereof, compounds of the formula I wherein
$R^1$ denotes -$(CH_2)_{3-4}$-NH - X, where
X = $C_1$-$C_6$-alkyl or a radical of the formula II;
$R^2$ and $R^3$ denote hydrogen;
$R^4$ denotes - CO-NH-$R^5$ and
Y denotes -$CH_2$-CO
being excluded.

**2.** Compound of the formula I according to Claim 1, characterised in that $R^1$ denotes -$CH_2$-$C_6H_4$-C(NH)-$NH_2$ or -$CH_2$-$C_6H_4$-$CH_2$-$NH_2$,
$R^2$ denotes H or $CH_3$,
Y denotes -$CH_2$-CO- and
$R^4$ denotes -CO-NH-$R^5$, wherein NH-$R^5$ represents an $\alpha$-amino acid radical, preferably a valine or phenylglycine radical.

**3.** Process for the preparation of a compound of the formula I according to Claim 1 or 2, by fragment condensation of, for example, compounds of the formula IIIa, IIIb or IIIc with compounds of the formula IV, wherein $R^1$, $R^2$, $R^3$, $R^4$, $X^1$ and m have the abovementioned meanings:

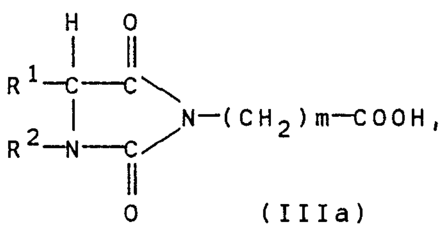
(IIIa)

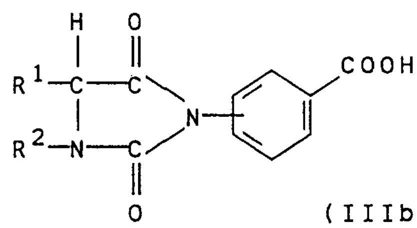
(IIIb)

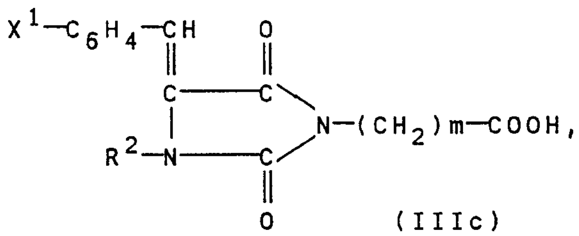
(IIIc)

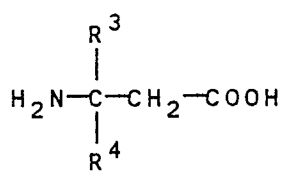
(IV)

**4.** Compound of the formula I according to Claim 1 or 2 for use as medicine.

**5.** Use of a compound of the formula I according to Claim 1 or 2 for the preparation of medicines for inhibition of platelet aggregation.

**6.** Use of a compound of the formula I according to Claim 1 or 2 for the preparation of medicines for inhibition of the bonding of osteoclasts to the bone surface.

**7.** Pharmaceutical formulation comprising a compound of the formula I according to Claim 1 or 2 or a physiologically tolerated salt thereof, and an acceptable excipient.

**Claims for the following Contracting States : ES, GR**

**1.** Process for preparing a compound of the formula I,

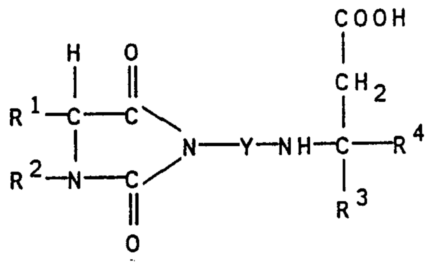

(I)

in which
Y denotes $-(CH_2)_m-CO-$ or

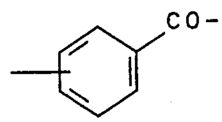

and m represents 1 - 4;
$R^1$ represents $-(CH_2)n-NH-X$, $-CH_2-C_6H_4-NH-X$, $-CH_2-C_6H_4-C(=NH)-NH_2$, $-CH_2-C_6H_4-CH_2-NH-X$ oder $-C_6H_4-NH-X$ or

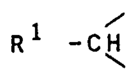

also denotes $>C=CH-C_6H_4-X^1$,
wherein n represents an integer from 3 to 5,
$X^1$ denotes $-CH_2NHX$, $-NHX$ oder $-C(=NH)-NH_2$,
X represents hydrogen or $C_1-C_6$-alkyl, or represents a radical of the formula II,

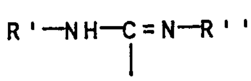

(II)

wherein
R' und R''independently of one another denote hydrogen or $C_1-C_6$-alkyl;
$R^2$ denotes hydrogen or $C_1-C_6$-alkyl;
$R^3$ denotes hydrogen or a phenyl radical;
$R^4$ denotes hydrogen, $COOR^5$, $CO-N(CH_3)-R^5$ or $CO-NH-R^5$, wherein
$R^5$ denotes hydrogen or $C_1-C_8$-alkyl, which is unsubstituted or mono- or polysubstituted by identical or different radicals $R^6$;
$R^6$ denotes hydroxy, carboxy, carboxamido, amino, mercapto, $C_1-C_8$-alkoxy, $C_1-C_{18}$-alkoxycarbonyl, phenyl-$C_1-C_3$-alkoxycarbonyl, $C_3-C_8$-cycloalkyl, halogen, nitro and trifluoromethyl, phenyl, phenyl-$C_1-C_8$-alkyl, $-NR^7R^8$, $-OR^7$, $-SR^7$, $-CONR^7R^8$, $-COOR^7$, $-COSR^7$, an amino acid side chain, a naturally occurring or unnatural amino acid radical, imino acid radical or a dipeptide radical as well as esters or amides thereof, it being possible for free functional groups optionally to be protected by protective groups which are customary in peptide chemistry;
$R^7$ denotes hydrogen, phenyl-$C_1-C_8$-alkyl, $C_1-C_{18}$-alkylcarbonyl, $C_1-C_8$-alkyloxycarbonyl, phenylcarbonyl, phenyl-$C_1-C_8$-alkylcarbonyl, phenyl-$C_1-C_8$-alkyloxycarbonyl which may be substituted by an amino group, a naturally occurring or unnatural amino acid radical, imino acid radical or a dipeptide radical;

$R^8$ denotes hydrogen, $C_1$-$C_8$-alkyl, phenyl or phenyl -$C_1$-$C_8$-alkyl and physiologically tolerated salts thereof, compounds of the formula I wherein
$R^1$ denotes -$(CH_2)_{3-4}$-NH - X, where
X = $C_1$-$C_6$-alkyl or a radical of the formula II;
$R^2$ and $R^3$ denote hydrogen;
$R^4$ denotes - CO-NH-$R^5$ and
Y denotes -$CH_2$-CO
being excluded, by fragment condensation of, for example, compounds of the formula IIIa, IIIb or IIIc with compounds of the formula IV, wherein $R^1$, $R^2$, $R^3$, $R^4$, $X^1$ and m have the abovementioned meanings:

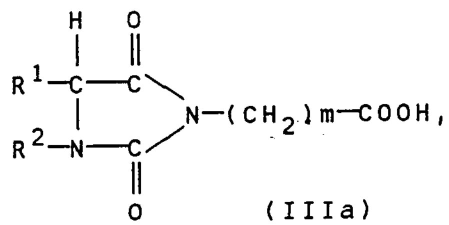
(IIIa)

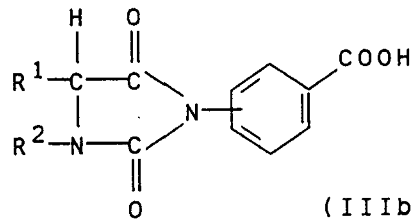
(IIIb)

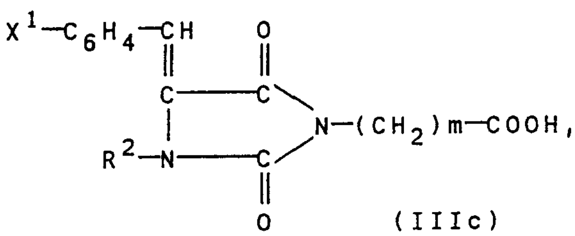
(IIIc)

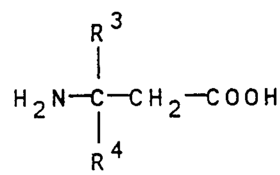
(IV)

**2.** Process according to Claim 1, characterised in that in formula I $R^1$ denotes -$CH_2$-$C_6H_4$-C(NH)-$NH_2$ or -$CH_2$-$C_6H_4$-$CH_2$-$NH_2$,
$R^2$ denotes H or $CH_3$,
Y denotes -$CH_2$-CO- and
$R^4$ denotes -CO-NH-$R^5$, wherein NH-$R^5$ represents an $\alpha$-amino acid radical, preferably a valine or phenylglycine radical.

**3.** Use of a compound of the formula I prepared according to Claim 1 and/or 2 for the preparation of medicines for inhibition of platelet aggregation.

**4.** Use of a compound of the formula I prepared according to Claim 1 and/or 2 for the preparation of medicines for inhibition of the bonding of osteoclasts to the bone surface.

**5.** Process for preparing a pharmaceutical formulation comprising a compound of the formula I prepared according to one of the processes of Claim 1 and/or 2 characterized in that it is rendered into a suitable form of administration together with an acceptable excipient.

## Revendications

**Revendications pour les Etats contractants suivants : AT, BE, CH, DE, DK, FR, GB, IT, LU, NL, SE**

**1.** Composé de formule I

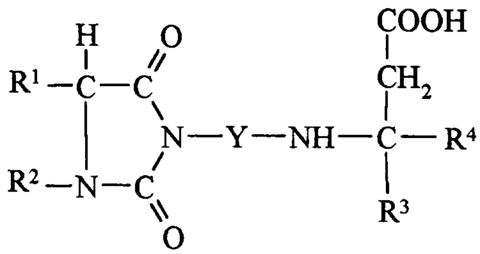

(I)

dans laquelle
Y représente - $(CH_2)_m$ - CO- ou

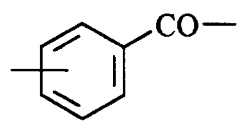

et m est 1-4;
$R^1$ représente $-(CH_2)_n$-NH-X, $-CH_2-C_6H_4$-NH-X, $-CH_2-C_6H_4-C(=NH)-NH_2$, $-CH_2-C_6H_4-CH_2$-NH-X ou $-C_6H_4$-NH-X,
ou bien

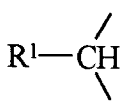

est aussi $>C=CH-C_6H_4-X^1$
où n représente un nombre entier de 3 à 5,
$X^1$ représente $-CH_2NHX$, -NHX ou $-C(=NH)-NH_2$,
X représente un hydrogène, un alkyle en $C_1$-$C_6$ ou un reste de formule II

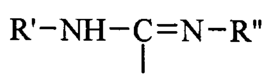

(II)

dans laquelle
R' et R" représentent indépendamment l'un de l'autre un hydrogène ou un alkyle en $C_1$-$C_6$;
$R^2$ représente un hydrogène ou un alkyle en $C_1$-$C_6$;
$R^3$ représente un hydrogène ou un reste phényle;
$R^4$ représente un hydrogène, $COOR^5$, $CO-N(CH_3)-R^5$ ou $CO-NH-R^5$, où
$R^5$ représente un hydrogène ou un alkyle en $C_1$-$C_8$ non substitué ou substitué une ou plusieurs fois par des restes $R^6$ identiques ou différents;
$R^6$ représente un reste hydroxy, carboxy, carboxamido, amino, mercapto, alcoxy en $C_1$-$C_8$, (alcoxy en $C_1$-$C_{18}$)carbonyle, phényl(alcoxy en $C_1$-$C_3$)carbonyle, cycloalkyle en $C_3$-$C_8$, halogène, nitro, trifluorométhyle, phényle, phényl(alkyle en $C_1$-$C_8$), $-NR^7R^8$, $-OR^7$, $-SR^7$, $-CONR^7R^8$, $-COOR^7$, $-COSR^7$, une chaîne latérale d'aminoacide, un reste d'aminoacide naturel ou non naturel, d'iminoacide ou d'un dipeptide, ainsi que de leurs esters et amides, les groupes fonctionnels libres pouvant éventuellement être protégés par les groupes protecteurs classiques de la chimie des peptides;

$R^7$ représente un hydrogène, un reste phényl(alkyle en $C_1$-$C_8$) éventuellement substitué par un groupe amino, (alkyl en $C_1$-$C_{18}$)carbonyle, (alkyl en $C_1$-$C_{18}$)oxycarbonyle, phénylcarbonyle, phényl(alkyl en $C_1$-$C_8$)carbonyle, phényl(alkyl en $C_1$-$C_8$)oxycarbonyle, ou un reste d'un aminoacide naturel ou non naturel, d'un iminoacide ou d'un dipeptide;
$R^8$ représente un hydrogène ou un reste alkyle en $C_1$-$C_8$, phényle ou phényl(alkyle en $C_1$-$C_8$);
et leurs sels physiologiquement acceptables, à l'exclusion des composés de formule I dans lesquels
$R^1$ est $-(CH_2)_{3-4}$-NH-X avec
X = un alkyle en $C_1$-$C_4$ ou un reste de formule II;
$R^2$ et $R^3$ représentent l'hydrogène;
$R^4$ est -CO-NH-$R^5$ et
Y représente $-CH_2$-CO.

**2.** Composé de formule I selon la revendication 1, caractérisé en ce que $R^1$ est $-CH_2-C_6H_4-C(NH)-NH_2$ ou $-CH_2-C_6H_4-CH_2-NH_2$,
$R^2$ est H ou $CH_3$,
Y est $-CH_2$-CO- et
$R^4$ est -CO-NH-$R^5$, NH-$R^5$ représentant un reste d'α-aminoacide, de préférence un reste de valine ou de phénylglycine.

**3.** Procédé de préparation d'un composé de formule I selon la revendication 1 ou 2 par condensation fragmentaire par exemple de composés de formule IIIa, IIIb ou IIIc avec des composés de formule IV, $R^1$, $R^2$, $R^3$, $R^4$, $X^1$ et m ayant les significations données ci-dessus:

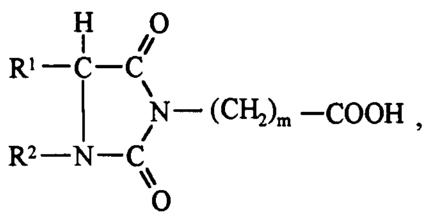

(IIIa)

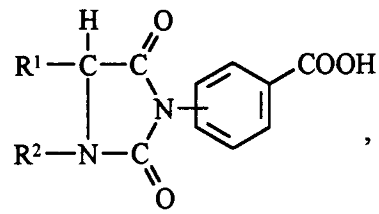

(IIIb)

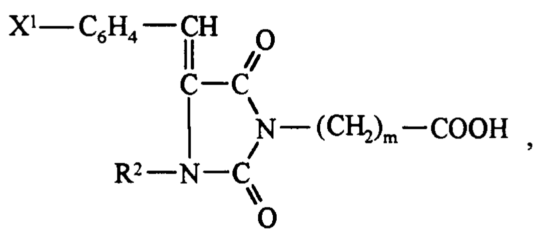

(IIIc)

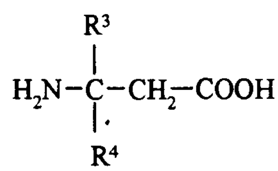

(IV)

**4.** Composé de formule I selon les revendications 1 ou 2 pour l'utilisation comme médicament.

**5.** Utilisation d'un composé de formule I selon les revendications 1 ou 2 pour la préparation de médicaments destinés à inhiber l'agrégation des thrombocytes.

**6.** Utilisation d'un composé de formule I selon les revendications 1 ou 2 pour la préparation de médicaments destinés à inhiber la fixation d'ostéoclastes sur la surface des os.

**7.** Composition pharmaceutique contenant un composé de formule I selon les revendications 1 ou 2 ou un de ses sels physiologiquement acceptables et un support acceptable.

**Revendications pour les Etats contractants suivants : ES, GR**

**1.** Procédé de préparation d'un composé de formule I

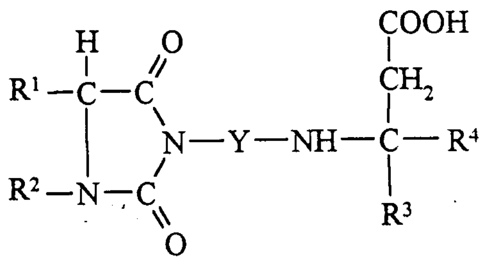

(I)

dans laquelle
Y représente - $(CH_2)m$ - CO- ou

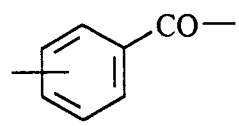

et m est 1-4;
$R^1$ représente $-(CH_2)_n$-NH-X, $-CH_2-C_6H_4$-NH-X, $-CH_2-C_6H_4-C(=NH)-NH_2$, $-CH_2-C_6H_4-CH_2$-NH-X ou $-C_6H_4$-NH-X,
ou bien

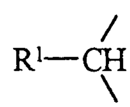

est aussi $>C=CH—C_6H_4—X^1$
où n représente un nombre entier de 3 à 5,
$X^1$ représente $-CH_2NHX$, -NHX ou $-C(=NH)-NH_2$,
X représente un hydrogène, un alkyle en $C_1$-$C_6$ ou un reste de formule II

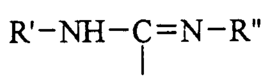

(II)

dans laquelle
R' et R" représentent indépendamment l'un de l'autre un hydrogène ou un alkyle en $C_1$-$C_6$;
$R^2$ représente un hydrogène ou un alkyle en $C_1$-$C_6$;
$R^3$ représente un hydrogène ou un reste phényle;
$R^4$ représente un hydrogène, $COOR^5$, $CO-N(CH_3)-R^5$ ou $CO-NH-R^5$, où
$R^5$ représente un hydrogène ou un alkyle en $C_1$-$C_8$ non substitué ou substitué une ou plusieurs fois par des restes $R^6$ identiques ou différents; $R^6$ représente un reste hydroxy, carboxy, carboxamido, amino, mercapto, alcoxy en $C_1$-$C_8$, (alcoxy en $C_1$-$C_{18}$)carbonyle, phényl(alcoxy en $C_1$-$C_3$)carbonyle, cycloalkyle en $C_3$-$C_8$, halogène, nitro, trifluorométhyle, phényle, phényl(alkyle en $C_1$-$C_8$), $-NR^7R^8$, $-OR^7$, $-SR^7$, $-CONR^7R^8$, $-COOR^7$, $-COSR^7$, une chaîne latérale d'aminoacide, un reste d'aminoacide naturel ou non naturel, d'iminoacide ou d'un dipeptide, ainsi que de leurs esters et amides, les groupes fonctionnels libres pouvant éventuellement être protégés par les groupes protecteurs classiques de la chimie des peptides;
$R^7$ représente un hydrogène, un reste phényl(alkyle en $C_1$-$C_8$) éventuellement substitué par un groupe amino, (alkyl en $C_1$-$C_{18}$)carbonyle, (alkyl en $C_1$-$C_{18}$)oxycarbonyle, phénylcarbonyle, phényl(alkyl en $C_1$-

$C_8$)carbonyle, phényl(alkyl en $C_1$-$C_8$)oxycarbonyle, ou un reste d'un aminoacide naturel ou non naturel, d'un iminoacide ou d'un dipeptide;
$R^8$ représente un hydrogène ou un reste alkyle en $C_1$-$C_8$, phényle ou phényl(alkyle en $C_1$-$C_8$);
et leurs sels physiologiquement acceptables, à l'exclusion des composés de formule I dans lesquels
$R^1$ est $-(CH_2)_{3-4}$-NH-X avec
X = un alkyle en $C_1$-$C_4$ ou un reste de formule II;
$R^2$ et $R^3$ représentent l'hydrogène;
$R^4$ est -CO-NH-$R^5$ et
Y représente $-CH_2$-CO,

par condensation fragmentaire par exemple de composés de formule IIIa, IIIb ou IIIc avec des composés de formule IV, $R^1$, $R^2$, $R^3$, $R^4$, $X^1$ et m ayant les significations données ci-dessus:

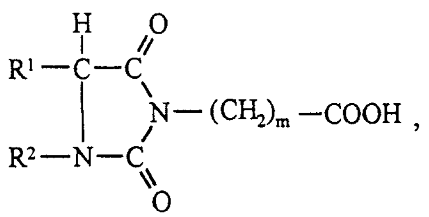

(IIIa)

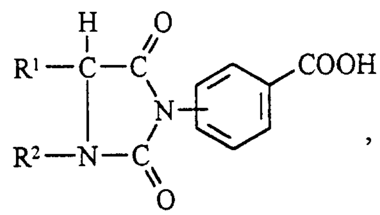

(IIIb)

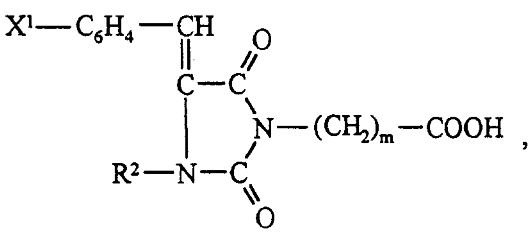

(IIIc)

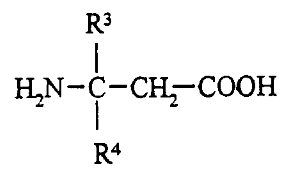

(IV)

**2.** Procédé selon la revendication 1 caractérisé en ce que, dans la formule I $R^1$ est $-CH_2-C_6H_4-C(NH)-NH_2$ ou $-CH_2-C_6H_4-CH_2-NH_2$,
$R^2$ est H ou $CH_3$.
Y est $-CH_2$-CO- et
$R^4$ est -CO-NH-$R^5$, NH-$R^5$ représentant un reste d'α-aminoacide, de préférence un reste de valine ou de phénylglycine.

**3.** Utilisation d'un composé de formule générale I préparé selon les revendications 1 et/ou 2 pour la préparation de médicaments destinés à inhiber l'agrégation des thrombocytes.

**4.** Utilisation d'un composé de formule générale I préparé selon les revendications 1 et/ou 2 pour la préparation de médicaments destinés à inhiber la fixation d'ostéoclastes sur la surface des os.

**5.** Procédé de préparation d'une composition pharmaceutique contenant un composé de formule I préparé selon l'un des procédés des revendications 1 et/ou 2, caractérisé en ce que l'on met ce composé avec un support acceptable sous une forme d'administration convenable.